# EUROPEAN PATENT APPLICATION

(11) **EP 2 199 412 A1**
(43) Date of publication of application: **23.06.2010**
(21) Application number: 08171266.3
(22) Date of filing: 10.12.2008
(51) Int. Cl.: C12Q 1/68

(54) **Compositions and methods for micro-RNA expression profiling of cancer stem cells**

(71) Applicant: Max-Planck-Gesellschaft zur Förderung der Wissenschaften e.V., 80539 München (DE)
(72) Inventor: Meister, Gunter, Dr., 82110 Germering (DE); Weinmann, Lasse, 81377 München (DE)
(74) Representative: Dilg, Haeusler, Schindelmann Patentanwaltsgesellschaft mbH

(57) **Abstract**

The present invention relates compositions and methods for microRNA expression profiling of cancer stem cells. In particular, the invention relates to a diagnostic kit of molecular markers for identifying one or more cancer stem cells, the kit comprising a plurality of nucleic acid molecules, each nucleic acid molecule encoding a microRNA sequence, wherein one or more of the plurality of nucleic acid molecules are differentially expressed in the tumor stem cells and in one or more control cells, and wherein the one or more differentially expressed nucleic acid molecules together represent a nucleic acid expression signature that is indicative for the presence of cancer stem cells. The invention further relates to methods using such nucleic acid expression signatures for identifying one or more cancer stem cells in a sample as well as for preventing the proliferation and/or self-renewal of such cancer stem cells. Finally, the invention is directed to a pharmaceutical composition for the prevention of the proliferation and/or self-renewal of cancer stem cells.

SEQUENCE LISTING

<110>   Max-Planck-Gesellschaft zur Förderung der Wissenschaften e.V.

<120>   Compositions and methods for micro-RNA expression profiling of
        cancer stem cells

<130>   M9774/SD

<140>   not assigned yet
<141>   2008-12-10

<160>   47

<170>   PatentIn version 3.3


<210>   1
<211>   23
<212>   RNA
<213>   Homo sapiens

<220>
<221>   misc_feature
<223>   hsa-miR-9 microRNA

<400>   1
ucuuugguua ucuagcugua uga                                              23


<210>   2
<211>   89
<212>   RNA
<213>   Homo sapiens

<220>
<221>   misc_feature
<223>   hsa-miR-9 microRNA precursor

<400>   2
cgggguuggu uguuaucuuu gguuaucuag cuguaugagu gguguggagu cuucauaaag      60
cuagauaacc gaaaguaaaa auaacccca                                        89


<210>   3
<211>   87
<212>   RNA
<213>   Homo sapiens

<220>
<221>   misc_feature
<223>   hsa-miR-9 microRNA precursor

<400>   3
ggaagcgagu uguuaucuuu gguuaucuag cuguaugagu guauuggucu ucauaaagcu      60
agauaaccga aaguaaaaac uccuuca                                          87


<210>   4
<211>   90
<212>   RNA
<213>   Homo sapiens

<220>
<221>   misc_feature
<223>   hsa-miR-9 microRNA precursor

<400>   4
ggaggcccgu uucucucuuu gguuaucuag cuguaugagu gccacagagc cgucauaaag      60

cuagauaacc gaaaguagaa augauucuca 90

<210> 5
<211> 22
<212> RNA
<213> Homo sapiens

<220>
<221> misc_feature
<223> hsa-miR-9* microRNA

<400> 5
auaaagcuag auaaccgaaa gu 22

<210> 6
<211> 89
<212> RNA
<213> Homo sapiens

<220>
<221> misc_feature
<223> hsa-miR-9* microRNA precursor

<400> 6
cgggguuggu uguuaucuuu gguuaucuag cuguaugagu gguguggagu cuucauaaag 60
cuagauaacc gaaaguaaaa auaacccca 89

<210> 7
<211> 87
<212> RNA
<213> Homo sapiens

<220>
<221> misc_feature
<223> hsa-miR-9* microRNA precursor

<400> 7
ggaagcgagu uguuaucuuu gguuaucuag cuguaugagu guauuggucu ucauaaagcu 60
agauaaccga aaguaaaaac uccuuca 87

<210> 8
<211> 90
<212> RNA
<213> Homo sapiens

<220>
<221> misc_feature
<223> hsa-miR-9* microRNA precursor

<400> 8
ggaggcccgu uucucucuuu gguuaucuag cuguaugagu gccacagagc cgucauaaag 60
cuagauaacc gaaaguagaa augauucuca 90

<210> 9
<211> 23
<212> RNA
<213> Homo sapiens

<220>
<221> misc_feature
<223> hsa-miR-17-5p microRNA

<400> 9
caaagugcuu acagugcagg uag 23

<210>   10
<211>   84
<212>   RNA
<213>   Homo sapiens

<220>
<221>   misc_feature
<223>   hsa-miR-17-5p microRNA precursor

<400>   10
gucagaauaa ugucaaagug cuuacagugc agguagugau augugcaucu acugcaguga      60
aggcacuugu agcauuaugg ugac                                              84


<210>   11
<211>   21
<212>   RNA
<213>   Homo sapiens

<220>
<221>   misc_feature
<223>   hsa-miR-106b microRNA

<400>   11
uaaagugcug acagugcaga u                                                 21


<210>   12
<211>   82
<212>   RNA
<213>   Homo sapiens

<220>
<221>   misc_feature
<223>   hsa-miR-106b microRNA precursor

<400>   12
ccugccgggg cuaaagugcu gacagugcag auaguggucc ucuccgugcu accgcacugu      60
ggguacuugc ugcuccagca gg                                                82


<210>   13
<211>   22
<212>   RNA
<213>   Homo sapiens

<220>
<221>   misc_feature
<223>   hsa-miR-15b microRNA

<400>   13
uagcagcaca ucaugguuua ca                                                22


<210>   14
<211>   98
<212>   RNA
<213>   Homo sapiens

<220>
<221>   misc_feature
<223>   hsa-miR-15b microRNA precursor

<400>   14
uugaggccuu aaaguacugu agcagcacau caugguuuac augcuacagu caagaugcga      60
aucauuauuu gcugcucuag aaauuuaagg aaauucau                               98

<210>   15
<211>   21
<212>   RNA
<213>   Homo sapiens

<220>
<221>   misc_feature
<223>   hsa-miR-151-5p microRNA

<400>   15
ucgaggagcu cacagucuag u                                                21


<210>   16
<211>   90
<212>   RNA
<213>   Homo sapiens

<220>
<221>   misc_feature
<223>   hsa-miR-151-5p microRNA precursor

<400>   16
uuuccugccc ucgaggagcu cacagucuag uaugucucau ccccuacuag acugaagcuc      60
cuugaggaca gggaugguca uacucaccuc                                       90


<210>   17
<211>   22
<212>   RNA
<213>   Homo sapiens

<220>
<221>   misc_feature
<223>   hsa-miR-320 microRNA

<400>   17
aaaagcuggg uugagagggc ga                                               22


<210>   18
<211>   82
<212>   RNA
<213>   Homo sapiens

<220>
<221>   misc_feature
<223>   hsa-miR-320 microRNA precursor

<400>   18
gcuucgcucc ccuccgccuu cucuucccgg uucuucccgg agucgggaaa agcuggguug      60
agagggcgaa aaaggaugag gu                                               82


<210>   19
<211>   21
<212>   RNA
<213>   Homo sapiens

<220>
<221>   misc_feature
<223>   hsa-miR-23b microRNA

<400>   19
aucacauugc cagggauuac c                                                21


<210>   20

<211>   97
<212>   RNA
<213>   Homo sapiens

<220>
<221>   misc_feature
<223>   hsa-miR-23b microRNA precursor

<400>   20
cucaggugcu cuggcugcuu ggguuccugg caugcugauu ugugacuuaa gauuaaaauc        60
acauugccag ggauuaccac gcaaccacga ccuuggc                                 97


<210>   21
<211>   22
<212>   RNA
<213>   Homo sapiens

<220>
<221>   misc_feature
<223>   hsa-miR-25 microRNA

<400>   21
cauugcacuu gucucggucu ga                                                 22


<210>   22
<211>   84
<212>   RNA
<213>   Homo sapiens

<220>
<221>   misc_feature
<223>   hsa-miR-25 microRNA

<400>   22
ggccaguguu gagaggcgga gacuugggca auugcuggac gcugcccugg gcauugcacu        60
ugucucgguc ugacagugcc ggcc                                               84


<210>   23
<211>   23
<212>   RNA
<213>   Homo sapiens

<220>
<221>   misc_feature
<223>   hsa-miR-191 microRNA

<400>   23
caacggaauc ccaaaagcag cug                                                23


<210>   24
<211>   92
<212>   RNA
<213>   Homo sapiens

<220>
<221>   misc_feature
<223>   hsa-miR-191 microRNA precursor

<400>   24
cggcuggaca gcgggcaacg gaaucccaaa agcagcuguu gucuccagag cauuccagcu        60
gcgcuuggau uucguccccu gcucuccugc cu                                      92


<210>   25
<211>   22

<212>   RNA
<213>   Homo sapiens

<220>
<221>   misc_feature
<223>   hsa-miR-15a microRNA

<400>   25
uagcagcaca uaaugguuug ug                                                22


<210>   26
<211>   83
<212>   RNA
<213>   Homo sapiens

<220>
<221>   misc_feature
<223>   hsa-miR-15a microRNA precursor

<400>   26
ccuuggagua aaguagcagc acauaauggu uuguggauuu ugaaaaggug caggccauau       60
ugugcugccu caaaaauaca agg                                               83


<210>   27
<211>   23
<212>   RNA
<213>   Homo sapiens

<220>
<221>   misc_feature
<223>   hsa-miR-103 microRNA

<400>   27
agcagcauug uacagggcua uga                                               23


<210>   28
<211>   78
<212>   RNA
<213>   Homo sapiens

<220>
<221>   misc_feature
<223>   hsa-miR-103 microRNA precursor

<400>   28
uacugcccuc ggcuucuuua cagugcugcc uuguugcaua uggaucaagc agcauuguac       60
agggcuauga aggcauug                                                     78


<210>   29
<211>   78
<212>   RNA
<213>   Homo sapiens

<220>
<221>   misc_feature
<223>   hsa-miR-103 microRNA precursor

<400>   29
uugugcuuuc agcuucuuua cagugcugcc uuguagcauu caggucaagc agcauuguac       60
agggcuauga aagaacca                                                     78


<210>   30
<211>   22
<212>   RNA

<213>   Homo sapiens

<220>
<221>   misc_feature
<223>   hsa-miR-16 microRNA

<400>   30
uagcagcacg uaaauauugg cg                                                22


<210>   31
<211>   89
<212>   RNA
<213>   Homo sapiens

<220>
<221>   misc_feature
<223>   hsa-miR-16 microRNA precursor

<400>   31
gucagcagug ccuuagcagc acguaaauau uggcguuaag auucuaaaau uaucuccagu       60
auuaacugug cugcugaagu aagguugac                                         89


<210>   32
<211>   81
<212>   RNA
<213>   Homo sapiens

<220>
<221>   misc_feature
<223>   hsa-miR-16 microRNA precursor

<400>   32
guuccacucu agcagcacgu aaauauuggc guagugaaau auauauuaaa caccaauauu       60
acugugcugc uuuaguguga c                                                 81


<210>   33
<211>   23
<212>   RNA
<213>   Homo sapiens

<220>
<221>   misc_feature
<223>   hsa-miR-221 microRNA

<400>   33
agcuacauug ucugcugggu uuc                                               23


<210>   34
<211>   110
<212>   RNA
<213>   Homo sapiens

<220>
<221>   misc_feature
<223>   hsa-miR-221 microRNA precursor

<400>   34
ugaacaucca ggucuggggc augaaccugg cauacaaugu agauuucugu guucguuagg       60
caacagcuac auugucugcu ggguuucagg cuaccuggaa acauguucuc                  110


<210>   35
<211>   21
<212>   RNA
<213>   Homo sapiens

<220>
<221>   misc_feature
<223>   hsa-miR-222 microRNA

<400>   35
agcuacaucu ggcuacuggg u                                                 21


<210>   36
<211>   110
<212>   RNA
<213>   Homo sapiens

<220>
<221>   misc_feature
<223>   hsa-miR-222 microRNA precursor

<400>   36
gcugcuggaa gguguaggua cccucaaugg cucaguagcc aguguagauc cugucuuucg       60
uaaucagcag cuacaucugg cuacuggguc ucugauggca ucuucuagcu                  110


<210>   37
<211>   21
<212>   RNA
<213>   Homo sapiens

<220>
<221>   misc_feature
<223>   hsa-miR-27a microRNA

<400>   37
uucacagugg cuaaguuccg c                                                 21


<210>   38
<211>   78
<212>   RNA
<213>   Homo sapiens

<220>
<221>   misc_feature
<223>   hsa-miR-27a microRNA precursor

<400>   38
   cugaggagca gggcuuagcu gcuugugagc aggguccaca ccaagucgug uucacagugg       60
   cuaaguuccg ccccccag                                                      78


<210>   39
<211>   22
<212>   RNA
<213>   Homo sapiens

<220>39
<221>   misc_feature
<223>   hsa-miR-21 microRNA

<400>   39
uagcuuauca gacugauguu ga                                                22


<210>   40
<211>   72
<212>   RNA
<213>   Homo sapiens

<220>

<221>   misc_feature
<223>   hsa-miR-21 microRNA precursor

<400>   40
ugucggguag cuuaucagac ugauguugac uguugaaucu cauggcaaca ccagucgaug      60
ggcugucuga ca                                                          72


<210>   41
<211>   22
<212>   RNA
<213>   Homo sapiens

<220>
<221>   misc_feature
<223>   hsa-miR-26a microRNA

<400>   41
uucaaguaau ccaggauagg cu                                               22


<210>   42
<211>   77
<212>   RNA
<213>   Homo sapiens

<220>
<221>   misc_feature
<223>   hsa-miR-26a microRNA precursor

<400>   42
guggccucgu ucaaguaauc caggauaggc ugugcagguc ccaaugggcc uauucuuggu      60
uacuugcacg gggacgc                                                     77


<210>   43
<211>   84
<212>   RNA
<213>   Homo sapiens

<220>
<221>   misc_feature
<223>   hsa-miR-26a microRNA precursor

<400>   43
ggcuguggcu ggauucaagu aauccaggau aggcuguuuc caucugugag gccuauucuu      60
gauuacuugu uucuggaggc agcu                                             84


<210>   44
<211>   21
<212>   RNA
<213>   Homo sapiens

<220>
<221>   misc_feature
<223>   hsa-miR-23a microRNA

<400>   44
aucacauugc cagggauuuc c                                                21


<210>   45
<211>   73
<212>   RNA
<213>   Homo sapiens

<220>
<221>   misc_feature

<223>   hsa-miR-23a microRNA precursor

<400>   45
ggccggcugg gguuccuggg gaugggauuu gcuuccuguc acaaaucaca uugccaggga      60
uuuccaaccg acc                                                          73


<210>   46
<211>   21
<212>   RNA
<213>   Homo sapiens

<220>
<221>   misc_feature
<223>   hsa-miR-27b microRNA

<400>   46
uucacagugg cuaaguucug c                                                 21


<210>   47
<211>   97
<212>   RNA
<213>   Homo sapiens

<220>
<221>   misc_feature
<223>   hsa-miR-27b microRNA

<400>   47
accucucuaa caaggugcag agcuuagcug auuggugaac agugauuggu uuccgcuuug      60
uucacagugg cuaaguucug caccugaaga gaaggug                                97

## Description

### FIELD OF THE INVENTION

The present invention relates to compositions and methods for microRNA (miRNA) expression profiling of cancer stem cells, particularly of stem cells derived from neuronal and/or glial tumors.

### BACKGROUND

Cancer still remains a major cause of death worldwide. Although a better understanding of the regulatory mechanisms underlying tumor etiology and progression has enabled increasing therapeutic success for some types of malignancy, for others (e.g., stomach cancer, pancreas cancer, glioblastoma) there has been little or almost no improvement in rates of long-term patient survival.

Cancerous cells develop from normal cells that gain the ability to proliferate aberrantly and to turn malignant. These malignant cells then grow clonally into tumors, eventually having the potential to metastasize. For decades, a central question in cancer biology has thus concerned the type of cells that can be transformed to form tumors.

Two important observations led to the hypothesis that so-called "cancer stem cells" sharing many characteristics with normal stem cells, including self-renewal and differentiation, may be responsible for growing and maintaining tumors. First, most tumors arise from a single cell, but not all the cells within a tumor are identical. This concept is also known as tumor heterogeneity (Park, C.H. et al. (1971) J. Natl. Cancer Inst. 46, 411-422). In fact, there are many different types of cells in a tumor; some are cancerous, whereas others are infiltrating normal cells that are thought to support the growth of the cancer cells. Second, it was demonstrated that a large number of cancer cells were required to grow a tumor (Bruce, W.R. and Van Der Gaag, H. (1963) Nature 199, 79-80; Hamburger, A.W. and Salmon, S.E. (1977) Science 197, 461-463).

These observations were seemingly at odds with the traditional stochastic model of cancer development predicting that every cancer cell has the potential to form a new tumor, but entry into the cell cycle is a stochastic event that occurs with low probability.

Nowadays, there is a burgeoning body of evidence suggesting that the regenerative capacity of tumors resides in only a small subpopulation of the total proliferative cells, namely "cancer stem cells" that have the exclusive ability to regenerate tumors (reviewed, e.g., in Lobo, N.A. et al. (2007) Annu. Rev. Cell Dev. Biol. 23, 675-699).

Tumors thus appear to be basically similar to normal tissues where a small population of self-renewing stem cells generates a larger population of transiently proliferative cells that eventually differentiates. Stem cell division in normal tissues is tightly related to functional needs and thus strictly controlled but this control is lost in malignancy and consequently there is ongoing expansion of the malignant tissue.

Hence, malignant stem cells could represent a target for therapeutic intervention but suitable approaches of how to eliminate them have not been available as these cancer stem cells appear to be protected by mechanisms that render them less susceptible to therapeutic killing (Al-Hajj, M. et al. (2004) Curr. Opin. Genet. Dev. 14, 43-47; Huff, C.A. et al. (2006) Blood 107, 431-434; Rich, J.N. (2007) Cancer Res. 67, 8980-8984). For example, the stem cell fractions of glioblastomas are radio-resistant and show increased DNA repair capacity and enrichment following radiation (Bao, S. et al. (2006) Nature 444, 756-760), whereas the stem cell fractions of malignant human breast cell lines selectively survive radiation (Philipps, T.M. et al. (2006) J. Natl. Cancer Inst. 98, 1777-1785).

Another unsolved issue with respect to the exploitation of the above-mentioned therapeutic concept concerns the reliable targeting of cancer stem cells. So far, no unambiguous molecular markers appear to exist for the identification of these cells. Bonnet and Dick isolated leukemic cancer stem cells based on the presence of CD34 proteins and a lack of CD38 proteins on their cell surface (Bonnet, D. and Dick, J.E. (1997). Nat. Med. 3, 730-737). In various brain tumors such as glioblastoma, tumorigenicity is restricted to cells expressing the cell surface marker CD133/prominin-1 (CD133-positive cells) (Singh, S.K. et al. (2003) Cancer Res. 63, 5821-5828), whereas the tumor-inducing abilities of breast cancers reside almost exclusively in a small CD44-positive fraction of total tumor cells (Al Hajj, M. et al. (2003) Proc. Natl. Acad. Sci. USA 100, 3983-3988). However, little is known about the cellular functions of any of these markers, particularly whether the expression of which is directly correlated with malignant tumor growth.

The identification of significant molecular markers for the detection of cancer stem cells would be of utmost clinical importance, particularly if these markers enable diagnosis and therapeutic targeting at an early stage of tumor progression in order to allow early stage treatment of cancers, particularly of carcinomas that are resistant to conventional therapy, while avoiding unnecessary surgical intervention.

Many diagnostic assays are also hampered by the fact that they are typically based on the analysis of only a single molecular marker, which might affect detection reliability and/or accuracy. In addition, a single marker normally does not enable detailed predictions concerning latency stages, tumor progression, and the like. Thus, there is still a continuing need for the identification of alternative molecular markers and assay formats overcoming these limitations.

One approach to address this issue might be based on small regulatory RNA molecules, in particular on microRNAs (miRNAs) which, constitute an evolutionary conserved class of endogenously expressed small non-coding RNAs of 20-25 nucleotides (nt) in size that can mediate the expression of target mRNAs and thus - since their discovery about ten years ago - have been implicated with critical functions in cellular development, differentiation, proliferation, and apoptosis.

MiRNAs are produced from primary transcripts that are processed to stem-loop structured precursors (pre-miRNAs) by the RNase III Drosha. After transport to the cytoplasm, another RNase III termed Dicer cleaves of the loop of the pre-miRNA hairpin to form a short doublestranded (ds) RNA intermediate, one strand of which is incorporated as mature miRNA into a miRNA-protein complex (miRNP). The miRNA guides the miRNPs to their target mRNAs where they exert their function (reviewed, e.g. in Bartel, D.P. (2004) Cell 23, 281-292; He, L. and Hannon, G.J. (2004) Nat. Rev. Genet. 5, 522-531). Depending on the degree of complementarity between the miRNA and its target, miRNAs can guide different regulatory processes. Target mRNAs that are highly complementary to miRNAs are specifically cleaved by mechanisms identical to RNA interference (RNAi) and the miRNAs function as short interfering RNAs (siRNAs). Target mRNAs with less complementarity to miRNAs are either directed to cellular degradation pathways and/or are translationally repressed. However, the mechanism of how miRNAs repress translation of their target mRNAs is still a matter of controversy.

Emerging data indicate that dysregulation of miRNA expression may *inter alia* be associated with the development and/or progression of certain types of cancer. In fact, it has been speculated based on cancer-associated alterations in miRNA expression and the observation that miRNAs are frequently located at genomic regions involved in cancers, and their gene regulatory function that miRNAs may act as both tumor suppressors and oncogenes (reviewed, e.g., in Esquela-Kerscher, A. and Slack, F.J (2006) Nat. Rev. Cancer 6, 259-269; Calin, G.A. and Croce, C.M. (2007) J. Clin. Invest. 117, 2059-2066; Blenkiron, C. and Miska, E.A. (2007) Hum. Mol. Genet. 16, R106-R113).

More systematic bead-based flow cytometric miRNA expression analyses revealed a global miRNA regulation in tumors indicating that miRNA profiling of host cells might indeed be suitable for cancer diagnosis (cf., e.g., Lu J. et al. (2005) Nature 435, 834-838) and various miRNAs whose expression appears characteristic for a particular tumor have been identified (Calin, G.A. and Croce, C.M. (2007), *supra*). However, to date only few of these aberrantly expressed miRNAs have been directly linked with clinically relevant prognostic factors for tumor development and/or progression.

Currently, only very few studies on miRNA expression analysis in stem cells are available. US patent application US 2008/0050722 Al discloses a subset of 17 miRNA molecules isolated from human embryonic stem cells. Stroncek and coworkers (Jin, P. et al. (2008) *J. Transl. Med.* **6**, 39) have recently described a different miRNA expression profile of blood hematopoietic stem cells that is distinct from the miRNA profile obtained in peripheral blood monocytes. However, no such miRNA signatures have been generated for any type of cancer stem cells.

Thus, there still remains a need for reliable molecular diagnostic markers that enable the rapid, reliable and cost-saving identification of cancer stem cells. In addition, there is also a continuing need for corresponding methods both for the identification and for preventing the proliferation and/or self-renewal of said cancer.

Accordingly, it is an object of the invention to provide such compositions and methods.

### SUMMARY OF THE INVENTION

In a first aspect, the present invention relates to a diagnostic kit of molecular markers for identifying one or more cancer stem cells, the kit comprising a plurality of nucleic acid molecules, each nucleic acid molecule encoding a microRNA sequence, wherein one or more of the plurality of nucleic acid molecules are differentially expressed in the cancer stem cells and in one or more control cells, and wherein the one or more differentially expressed nucleic acid molecules together represent a nucleic acid expression signature that is indicative for the presence of cancer stem cells.

In preferred embodiments, the cancer stem cells are CD133-positive, and the control cells are CD133-negative cancer cells.

In other preferred embodiments, the cancer stem cells are derived from neuronal and/or glial tumors, particularly preferably from gliobastoma.

In further specific embodiments, the nucleic acid expression signature comprises at least three, preferably at least five, and more preferably at least eight nucleic acid molecules.

In particularly preferred embodiments, the nucleic acid expression signature comprises nucleic acid molecules encoding microRNA sequences selected from the group consisting of hsa-miR-9 and hsa-miR-9*. Preferably, the expression of any of the nucleic acid molecules encoding hsa-miR-9 and hsa-miR-9* is up-regulated in the one or more cancer stem cells compared to the one or more control cells.

In other specific embodiments, the nucleic acid expression signature further comprises any one or more nucleic acid molecules encoding microRNA sequences selected from the group consisting of hsa-miR-17-5p, hsa-miR-106b, hsa-miR-15b, hsa-miR-151-5p, hsa-miR-320, hsa-miR-23b, hsa-miR-25, hsa-miR-191, hsa-miR-15a, hsa-miR-103, hsa-miR-16, hsa-miR-221, hsa-miR-222, hsa-miR-27a, hsa-miR-21, hsa-miR-26a, hsa-miR-23a, and hsa-miR-27b.

Preferably, the expression of any of the nucleic acid molecules encoding hsa-miR-17-5p, hsa-miR-106b, hsa-miR-15b, hsa-miR-151-5p, hsa-miR-320, hsa-miR-23b, hsa-miR-25, hsa-miR-191, hsa-miR-15a, hsa-miR-103, and hsa-miR-16 is up-regulated and the expression of any of the nucleic acid molecules hsa-miR-221, hsa-miR-222, hsa-miR-27a, hsa-miR-21, hsa-miR-26a, hsa-miR-23a, and hsa-miR-27b is down-regulated in the in the one or more cancer stem cells compared to the one or more control cells.

In further particularly preferred embodiments, the nucleic acid expression signature comprises nucleic acid molecules encoding hsa-miR-9, hsa-miR-9*, hsa-miR-17-5p, hsa-miR-106b, hsa-miR15b, hsa-miR-221, hsa-miR-222, hsa-miR-27a, and hsa-miR-21.

In a second aspect, the present invention relates to a method for identifying one or more cancer stem cells, preferably CD133-positive cancer stem cells, the method comprising: (a) determining in the one or more cancer stem cells the expression levels of a plurality of nucleic acid molecules, each nucleic acid molecule encoding a microRNA sequence, (b) determining the respective expression levels of the plurality of nucleic acid molecules in one or more control cells, and (c) identifying from the plurality of nucleic acid molecules one or more nucleic acid molecules that are differentially expressed in the target and control cells by comparing the respective expression levels obtained in steps (a) and (b), wherein the one or more differentially expressed nucleic acid molecules together represent a nucleic acid expression signature, as defined in any of claims 1 to 9, that is indicative for the presence of cancer stem cells.

In preferred embodiments, the cancer stem cells are CD133-positive and the control cells are CD133-negative cancer cells, and the method further comprises separating the CD133-positive and CD133-negative cells prior to performing step (a).

In a third aspect, the present invention relates to a method for preventing the proliferation and/or self-renewal of one or more cancer stem cells, preferably CD133-positive cancer stem cells, the method comprising: (a) identifying in the one or more cancer stem cells a nucleic acid expression signature by using a method as defined herein, and (b) modifying in the one or more cancer stem cells the expression of one or more nucleic acid molecules encoding a microRNA sequence that is/are comprised in the nucleic acid expression signature in such way that the expression of a nucleic acid molecule whose expression is up-regulated in the one or more cancer stem cells is down-regulated and the expression of a nucleic acid molecule whose expression is down-regulated in the one or more cancer stem cells is up-regulated.

In a forth aspect, the present invention relates to a pharmaceutical composition for preventing the proliferation and/or self-renewal of one or more cancer stem cells, preferably CD133-positive cancer stem cells, the composition comprising one or more nucleic acid molecules, each nucleic acid molecule encoding a sequence that is at least partially complementary to a microRNA sequence encoded by a nucleic acid molecule whose expression is up-regulated in the one or more cancer stem cells, as defined herein, and/or that corresponds to a microRNA sequence encoded by a nucleic acid molecule whose expression is down-regulated in the one or more cancer stem cells, as defined herein.

In preferred embodiments, the cancer stem cells are derived from neuronal and/or glial tumors, preferably from gliobastoma.

Finally, in a fifth aspect, the present invention relates to the use of said pharmaceutical composition for the manufacture of a medicament for the prevention and/or treatment of cancer, preferably of neuronal and/or glial cancers.

### DESCRIPTION OF THE DRAWINGS

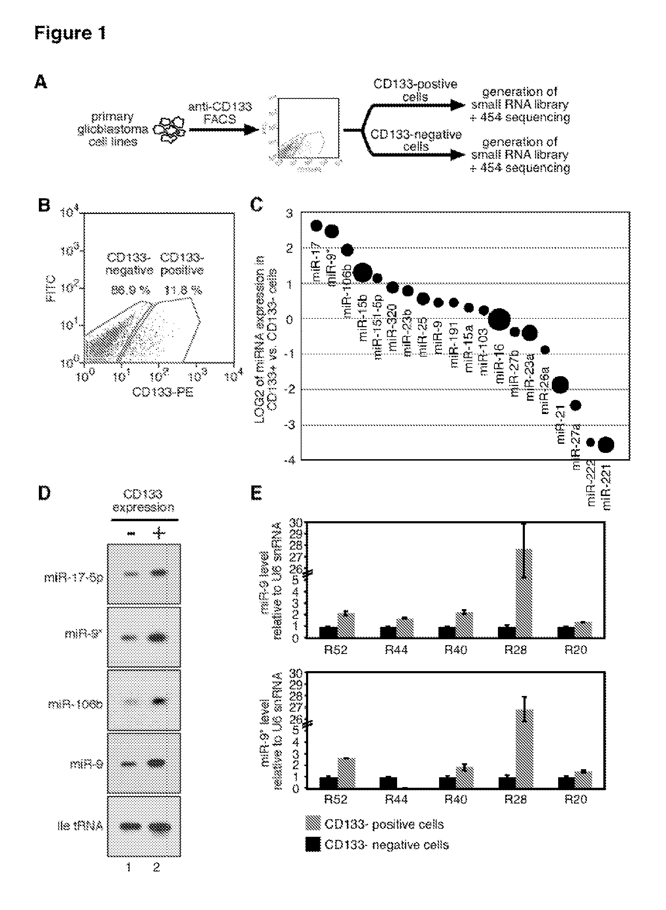

**FIGURE 1****: miRNA expression profiles of CD133-positive and CD133-negative glioblastoma cell fractions.**
   **1(A)** Primary human glioblastoma cell lines were incubated with an anti-CD133-phycoerythrin (PE) antibody and separated by fluorescence-assisted cell sorting (FACS) into CD133-positive and CD133-negative cell fractions. RNA was isolated and used to generate small RNA libraries, followed by 454 pyro-sequencing.
   **1(B)** depicts a typical FACS profile of the primary glioblastoma cell line R11. The dot-plot shows CD133-PE fluorescence on the x-axis and fluorescein isothiocyanate (FITC) on the y-axis as a control for autofluorescence. The regions indicate CD133-negative and CD133-positive cells, respectively.
   **1(C)** is a representation of the most abundant miRNAs in the libraries of CD133-negative and CD133-positive cells. The y-axis shows log2 values of the relative miRNA abundance in CD133-positive cells vs. CD133-negative cells. Thus, miRNAs that are enriched in CD133-positive cells are displayed above the 0-axis. The size of the dots corresponds to the abundance of each miRNA in both libraries.
   **1(D)** The R28 primary glioblastoma cell line was separated into CD133-positive and CD133-negative cell fractions as in Fig. 1(A). RNA was extracted and analyzed by Northern Blotting for the presence of miRNAs that were enriched in CD133-positive cells according to Fig. 1(C). U6 snRNA was used as loading control.
   **1(E)** Primary glioblastoma cell lines were separated as in Fig. 1(A) and analyzed for the presence of hsa-miR-9 (upper panel) and hsa-miR-9* (lower panel) by quantitative PCR (qPCR). miRNA levels were normalized to U6 snRNA levels, and values for CD133-negative cell fractions were normalized to 1.

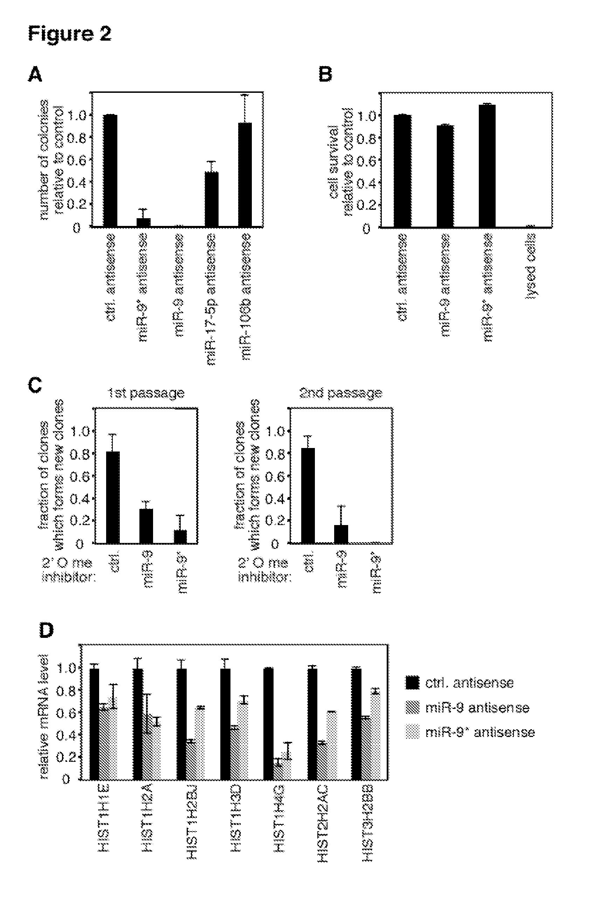

**FIGURE 2****: Inhibition of hsa-miR-9 and hsa-miR-9* impairs self-renewal and proliferation of human glioblastoma cells.**
   **2(A)** R11 cells were transfected twice with antagomirs and seeded to 48 well plates. Neurosphere-like colonies were counted 4 weeks after transfection.
   **2(B)** R11 cells were transfected twice with antagomirs, and cell survival was assessed by measuring lactate dehydrogenase (LDH) activity in the supernatant 48 hours post transfection. Cell lysis with 1% Triton X-100 was used as positive control for cytotoxicity.
   **2(C)** Left panel: Single colonies of R11 cells that grew after treatments in Fig. 2(A) were picked, trypsinized and passaged to wells in a 48 well plate. Trypsinization efficiency was monitored by light microscopy. Cells were grown for 10 days, and the fraction of clones which formed new clones was quantified. Right panel: The colonies quantified in the left panel were picked and passaged again, as described above.
   **2(D)** R11 cells were transfected with antagomirs, RNA was isolated and the expression of the indicated histone genes was assessed by qPCR. mRNA levels were normalized to GAPDH mRNA levels and to control samples.

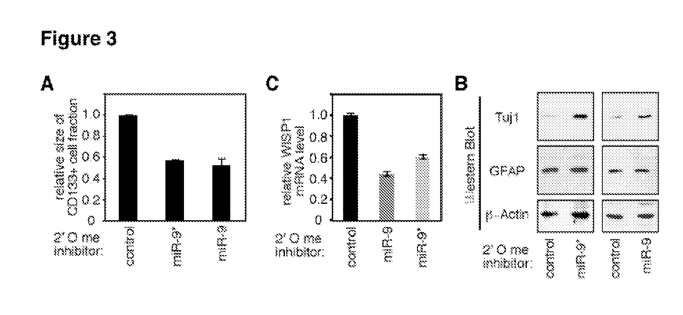

**FIGURE 3****: Inhibition of hsa-miR-9 and hsa-miR-9* reduces the pool of CD133-positive glioma cells, enhances the expression of the neuronal marker protein Tuj1 and reduces expression of the Wnt target gene WISP1.**
   **3(A)** R11 cells were transfected with antagomirs for 2 days and analyzed for CD133 expression by flow cytometry. The figure shows the size of the CD133-positive cell fractions relative to the control sample.
   **3(B)** R11 cells were transfected with antagomirs for 2 days, lysed and analyzed for the expression of neuronal class III β-tubulin (Tuj1) and glial fibrillary acidic protein (GFAP) by Western blotting. β-Actin was used as loading control.
   **3(C)** R11 cells were transfected with antagomirs for 2 days. RNA was isolated, and levels of WNT1-inducible signaling pathway protein 1 (WISP1) mRNA were quantified relative to GAPDH mRNA by qPCR.

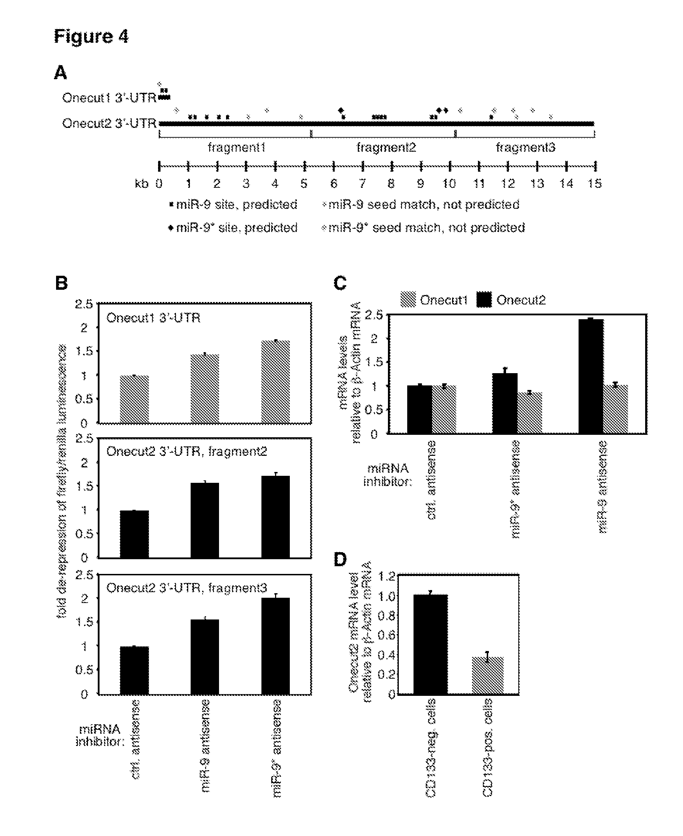

**FIGURE 4****: hsa-miR-9 and hsa-miR-9* repress the expression of Onecut1 and Onecut2.**
   **4(A)** A schematic representation of the Onecut1 and Onecut2 3'-UTRs with miRNA seed matches, which are predicted or not predicted as miRNA binding sites.
   **4(B)** T98G human glioma cells were co-transfected with antagomirs and dual luciferase reporter constructs carrying the indicated 3'-UTRs fused to the firefly luciferase open reading frame (orf). Luminescence was measured 24 hrs post transfection, and firefly/*Renilla* ratios were normalized to control antagomir and to control plasmid transfections.
   **4(C)** R11 cells were transfected with antagomirs for 2 days. RNA was isolated, and Onecut1 and Onecut2 mRNA expression was quantified by qPCR relative to β-Actin mRNA levels.
   **4(D)** Onecut2 mRNA levels in CD133-positive cells, relative to CD133-negative cells and to β-actin mRNA.

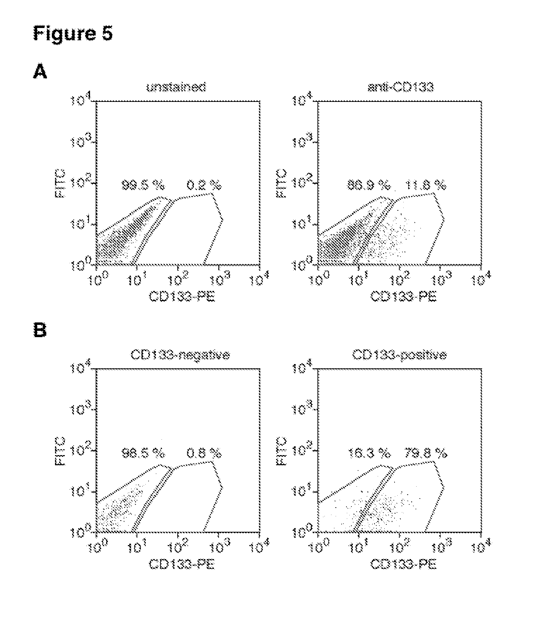

**FIGURE 5****: CD133-positive and CD133-negative cells are efficiently separated by flow cytometry.**
   R11 cells were analyzed by flow cytometry as shown in Fig. 1 (A)/(B).
   **5(A)** Flow cytometry analysis of unstained cells (left panel) and cells stained with anti-CD133-PE (right panel).
   **5(B)** After FACS separation, CD133-negative (left panel) and CD133-positive cell fractions (right panel) were re-analyzed by flow cytometry.
**FIGURE 6****: hsa-miR-9 and hsa-miR-9* can be independently depleted by using 2'O methyl antisense oligonucleotides.**
   R11 cells were transfected twice with antagomirs. RNA was isolated and miRNA levels were analyzed by qPCR relative to U6 RNA expression and to control antisense-transfected samples.

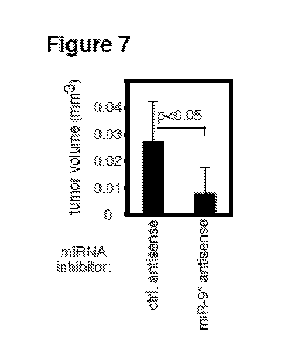

**FIGURE 7****: Inhibition of hsa-miR-9* reduces glioblastoma growth *in vivo.***
   R11 cells were transfected twice with either control or hsa-miR-9* antisense oligonucleotides and intracranially injected 1 day after the second transfection into T-lymphocyte-deficient NMRI(nu/nu) mice. Equal cell numbers (200.000 cells per mouse) were used for the second transfection. Mice were sacrificed 12 weeks after injection, and tumor volumes were determined from a series of consecutive tumor tissue sections using hematoxylin and eosin (H&E) staining for each mouse. Effects were statistically significant (p = 0.04, one-sided Student's t-test)

### DETAILED DESCRIPTION OF THE INVENTION

The present invention is based on the unexpected finding that cancer stem cells can be reliably identified based on a particular miRNA expression signature both with high accuracy and sensitivity. In addition, modifying the expression of one or more miRNAs comprised in the signature defined herein represents a promising target for therapeutic intervention in order to prevent proliferation and/or self-renewal of said cancer stem cells and/or to promote their differentiation during tumor progression. This, in turn, allows the detection of a cancerous condition at an early disease state as well as offers a therapeutic approach for treating tumors not susceptible to conventional treatment.

The present invention illustratively described in the following may suitably be practiced in the absence of any element or elements, limitation or limitations, not specifically disclosed herein. The present invention will be described with respect to particular embodiments and with reference to certain drawings but the invention is not limited thereto but only by the claims. The drawings described are only schematic and are to be considered non-limiting.

Where the term "comprising" is used in the present description and claims, it does not exclude other elements or steps. For the purposes of the present invention, the term "consisting of' is considered to be a preferred embodiment of the term "comprising ". If hereinafter a group is defined to comprise at least a certain number of embodiments, this is also to be understood to disclose a group, which preferably consists only of these embodiments.

Where an indefinite or definite article is used when referring to a singular noun e.g. "a", "an" or "the", this includes a plural of that noun unless specifically stated otherwise.

In case, numerical values are indicated in the context of the present invention the skilled person will understand that the technical effect of the feature in question is ensured within an interval of accuracy, which typically encompasses a deviation of the numerical value given of ± 10%, and preferably of ± 5%.

Furthermore, the terms first, second, third, (a), (b), (c), and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used. The following terms or definitions are provided solely to aid in the understanding of the invention. These definitions should not be construed to have a scope less than understood by a person of ordinary skill in the art.

In a first aspect, the present invention relates to a diagnostic kit of molecular markers for identifying one or more cancer stem cells, the kit comprising a plurality of nucleic acid molecules, each nucleic acid molecule encoding a microRNA sequence, wherein one or more of the plurality of nucleic acid molecules are differentially expressed in the cancer stem cells and in one or more control cells, and wherein the one or more differentially expressed nucleic acid molecules together represent a nucleic acid expression signature that is indicative for the presence of cancer stem cells.

The term "cancer" (also referred to as "carcinoma"), as used herein, generally denotes any type of malignant neoplasm, that is, any morphological and/or physiological alterations (based on genetic re-programming) of target cells exhibiting or having a predisposition to develop characteristics of a carcinoma as compared to unaffected (healthy) wild-type control cells. Examples of such alterations may relate *inter alia* to cell size and shape (enlargement or reduction), cell proliferation (increase in cell number), cell differentiation (change in physiological state), apoptosis (programmed cell death) or cell survival.

The term "cancer stem cells" (also referred to as target cells), as used herein, refers to a (sub-) population of cancer cells that have the exclusive ability to regenerate tumors, that is, cells are responsible for tumor maintenance and metastasis. Cancer stem cells share many characteristics with normal stem cells, including self-renewal and differentiation. The term "self-renewal" concerns a specific mitotic cell division that enables a stem cell to produce one (asymmetrical) or two (symmetrical) daughter stem cell with essentially the same development and replication potential. The ability to self-renew enables expansion of the stem cell compartment in response to systemic or local signals, which trigger massive proliferation and maintenance of a tissue-specific undifferentiated pool of cells in an organ or tissue. The term "differentiation" refers to the process of stem or progenitor cells activating genetic and epigenetic mechanisms to define the specialized characteristics of multiple types of mature cells. Thus, differentiation involves the production of (daughter) cells that become tissue-specific specialized cells.

The cancer stem cells employed in the present invention may be of human or non-human origin. However, the invention is typically performed with human cells. The term "one or more cells", as used herein, is to be understood not only to include individual cells but also tissues, organs, and organisms. The cancer stem cells used herein may be derived from any type of tumor including neuronal and/or glial, colon, lung, liver, pancreatic, prostate, head and neck, and renal cancers.

In preferred embodiments, the cancer stem cells are derived from neuronal and/or glial tumors, particularly preferably from gliobastoma. The term "neuronal and/or glial tumor", as used herein, denotes any type of cancer of the central nervous system including any form of brain tumors. Examples of neuronal and/or glial tumors include *inter alia* neuroblastoma, ependymal tumors, neuroectodermal tumors including medulloblastoma, grade 1-3 astrocytoma, and gliobastoma (also referred to as "grade 4 astrocytoma"), with the latter one being particularly preferred.

"Glioblastoma" (also referred to "glioblastoma multiforme") represents the most common and most aggressive type of primary brain tumor, accounting for about 50% of all primary brain tumor cases and 20% of all intracranial tumors. Despite being the most prevalent form of primary brain tumor, gliobastoma occurs in only 2-3 cases per 100.000 people in Europe and North America. The most prevalent symptom of glioblastoma is a progressive memory, personality, or neurological deficit due to temporal and frontal lobe involvement. Glioblastoma is characterized by the presence of small areas of necrotizing surrounded by anaplastic cells (pseudopalisading necrosis). This characteristic, as well as the presence of hyperplastic blood vessels, distinguishes glioblastoma from grade 3 astrocytoma.

Currently, no curative treatment for glioblastoma is available, but only palliative (i.e. life-prolonging) measures. This is mainly due to the high resistance of tumor cells to radio- or chemotherapy, the very limited capacity of the brain to repair itself, and the failure of many drugs to cross the blood-brain-barrier. The median survival time from the time of diagnosis without any treatment is only 3 months that can be prolonged by palliative measures (e.g., surgery) up to three years.

In further preferred embodiments, the cancer stem cells are CD133-positive cells, that is cells expressing on their surface the CD133 protein (also known as prominin-1), a 5-transmembrane glycoprotein. Little is known about the cellular function of CD133, even though it has been speculated that CD133 may be involved in regulating tumor vasculature (Hilbe, W. et al. (2004) J. Clin. Pathol. 57, 965-969). In different brain tumors, cancer stem cells are found exclusively in the fraction of CD133-positive (CD133⁺) cells (Singh, S.K. et al. (2003), *supra*). Recently, CD133 has also been demonstrated to be expressed in cancer stem cells derived from leukemia, retinoblastoma, renal tumors, pancreatic tumors, colon carcinoma, prostate carcinoma, and hepatocellular carcinoma (see, e.g., Liu, G. et al. (2006) Mol. Cancer 5, 67; O'Brien, C.A. et al. (2007) Nature 445, 106-110; Ricci-Vtiani, L. et al. (2007) Nature 445, 111-115; Ma, S. et al. (2007) Gastroenterology 132, 2542-2556; Chen, Y.C. et al. (2008) PLoS ONE 3, e2637, and the references cited therein).

The term "control cells", as used herein, may refer to (healthy) wild-type cells not having characteristics of any cancerous phenotype. Typically, however, the term "control cells" denotes tumor cells (preferably derived from the same sample as the cancer stem cells employed) not belonging to the (sub-)population of cancer stem cells, that is, control cells are cancer cells not having the capability of self-renewal and differentiation common to stem cells. In preferred embodiments, the cancer stem cells are CD133-positive and the control cells CD133-negative (CD133⁻) cancer cells.

Typically, the cancer stem cells and the control cells used are derived from biological samples collected from the subjects to be diagnosed for the presence or the predisposition to develop cancer, preferably a neuronal and/or glial cancer, particularly preferably glioblastoma. The biological samples may include body tissues and fluids, such as blood, sputum, cerebrospinal fluid, and urine. Furthermore, the biological sample may contain a cell population or a cell extract derived from a tissue suspected to be cancerous, preferably derived from a neuronal and/or glial tissue. The samples to be analyzed herein are typically biopsies or resections. If applicable, the cells may be purified from the body tissues and fluids prior to use. According to the present invention, the expression levels of the nucleic acid molecules of the present invention are determined in the subject-derived biological sample(s). The sample(s) used for detection in the *in vitro* methods of the present invention should generally be collected in a clinically acceptable manner, preferably in a way that nucleic acids (in particular RNA) are preserved.

The term "microRNA" (or "miRNA"), as used herein, is given its ordinary meaning in the art (reviewed, e.g. in Bartel, D.P. (2004) Cell 23, 281-292; He, L. and Hannon, G.J. (2004) Nat. Rev. Genet. 5, 522-531). Accordingly, a "microRNA" denotes a RNA molecule derived from a genomic locus that is processed from transcripts that can form local RNA precursor miRNA structures. The mature miRNA is usually 20, 21, 22, 23, 24, or 25 nucleotides in length, although other numbers of nucleotides may be present as well, for example 18, 19, 26 or 27 nucleotides.

The miRNA encoding sequence has the potential to pair with flanking genomic sequences, placing the mature miRNA within an imperfect RNA duplex (herein also referred to as stem-loop or hairpin structure or as pre-miRNA), which serves as an intermediate for miRNA processing from a longer precursor transcript. This processing typically occurs through the consecutive action of two specific endonucleases termed Drosha and Dicer, respectively. Drosha generates from the primary transcript (herein also denoted "pri-miRNA") a miRNA precursor (herein also denoted "pre-miRNA") that typically folds into a hairpin or stem-loop structure. From this miRNA precursor a miRNA duplex is excised by means of Dicer that comprises the mature miRNA at one arm of the hairpin or stem-loop structure and a similar-sized segment (commonly referred to miRNA*) at the other arm. The miRNA is then guided to its target mRNA to exert its function, whereas the miRNA* is degraded in most cases. In addition, miRNAs are typically derived from a segment of the genome that is distinct from predicted protein-coding regions. Alternatively, miRNAs can derive from introns of protein-coding genes.

The term "miRNA precursor" (or "precursor miRNA" or "pre-miRNA"), as used herein, refers to the portion of a miRNA primary transcript from which the mature miRNA is processed. Typically, the pre-miRNA folds into a stable hairpin (i.e. a duplex) or a stem-loop structure. The hairpin structures range from 50 to 120 nucleotides in length, typically from 55 to 100 nucleotides in length, and preferably from 60 to 90 nucleotides in length (counting the nucleotide residues pairing to the miRNA (i.e. the "stem") and any intervening segment(s) (i.e. the "loop") but excluding more distal sequences).

The term "nucleic acid molecule encoding a microRNA sequence", as used herein, denotes any nucleic acid molecule coding for a microRNA (miRNA). Thus, the term does not only refer to mature miRNAs but also to the respective precursor miRNAs and primary miRNA transcripts as defined above. Furthermore, the present invention is not restricted to RNA molecules but also includes corresponding DNA molecules encoding a microRNA, e.g. DNA molecules generated by reverse transcribing a miRNA sequence. A nucleic acid molecule encoding a microRNA sequence according to the invention typically encodes a single miRNA sequence (i.e. an individual miRNA). However, it is also possible that such nucleic acid molecule encodes two or more miRNA sequences (i.e. two or more miRNAs), for example a transcriptional unit comprising two or more miRNA sequences under the control of common regulatory sequences such as a promoter or a transcriptional terminator.

In addition, the term "nucleic acid molecule encoding a microRNA sequence", as used herein, is also to be understood to include "sense nucleic acid molecules" (i.e. molecules whose nucleic acid sequence (5'→3') matches or corresponds to the encoded miRNA (5'→3') sequence) and "anti-sense nucleic acid molecules" (i.e. molecules whose nucleic acid sequence is complementary to the encoded miRNA (5'→3') sequence or, in other words, matches the reverse complement (3'→5') of the encoded miRNA sequence). The term "complementary", as used herein, refers to the capability of an "anti-sense" nucleic acid molecule sequence of forming base pairs, preferably Watson-Crick base pairs, with the corresponding "sense" nucleic acid molecule sequence (having a sequence complementary to the anti-sense sequence).

Within the scope of the present invention, two nucleic acid molecules (i.e. the "sense" and the "anti-sense" molecule) may be perfectly complementary, that is, they do not contain any base mismatches and/or additional or missing nucleotides. Alternatively, the two molecules comprise one or more base mismatches or differ in their total numbers of nucleotides (due to additions or deletions). Preferably, the "complementary" nucleic acid molecule comprises at least ten contiguous nucleotides showing perfect complementarity with a sequence comprised in corresponding "sense" nucleic acid molecule.

Accordingly, the plurality of nucleic acid molecules encoding a miRNA sequence that are comprised in a diagnostic kit of the present invention may include one or more "sense nucleic acid molecules" and/or one or more "anti-sense nucleic acid molecules". In case, the diagnostic kit includes one or more "sense nucleic acid molecules" (i.e. the miRNA sequences as such), said molecules are to be considered to constitute the totality or at least a subset of differentially expressed miRNAs (i.e. molecular markers) being indicative for the presence of cancer stem cells. On the other hand, in case a diagnostic kit includes one or more "anti-sense nucleic acid molecules" (i.e. sequences complementary to the miRNA sequences), said molecules may comprise *inter alia* probe molecules (for performing hybridization assays) and/or oligonucleotide primers (e.g., for reverse transcription or PCR applications) that are suitable for detecting and/or quantifying one or more particular (complementary) miRNA sequences in a given sample.

A plurality of nucleic acid molecules as defined within the present invention may comprise at least two, at least ten, at least 50, at least 100, at least 200, at least 500, at least 1.000, at least 10.000 or at least 100.000 nucleic acid molecules, each molecule encoding a miRNA sequence.

The term "differentially expressed", as used herein, denotes an altered expression level of a particular miRNA in the target cells as compared to the healthy control cells, which may be an up-regulation (i.e. an increased miRNA concentration in the target cells) or a down-regulation (i.e. a reduced or abolished miRNA concentration in the target cells). In other words, the nucleic acid molecule is activated to a higher or lower level in the target cells than in the control cells.

Within the scope of the present invention, a nucleic acid molecule is to considered differentially expressed if the respective expression levels of this nucleic acid molecule in target cells and control cells typically differ by at least 5% or at least 10%, preferably by at least 20% or at least 25%, and most preferably by at least 30% or at least 50%. Thus, the latter values correspond to an at least 1.3-fold or at least 1.5-fold up-regulation of the expression level of a given nucleic acid molecule in the target cells compared to the wild-type control cells or *vice versa* an at least 0.7-fold or at least 0.5-fold down-regulation of the expression level in the target cells, respectively.

The term "expression level", as used herein, refers to extent to which a particular miRNA sequence is expressed from its genomic locus, that is, the concentration of a miRNA in the one or more cells (cancer stem cells or control cells) to be analyzed.

The determining of expression levels typically follows established standard procedures known in the art (cf., for example, Sambrook, J. et al. (1989) Molecular Cloning: A Laboratory Manual. 2nd Ed., Cold Spring Harbor Laboratory Press, Cold Spring Harbor, NY; Ausubel, F.M. et al. (2001) Current Protocols in Molecular Biology. Wiley & Sons, Hoboken, NJ). Determination may occur at the RNA level, for example by Northern blot analysis using miRNA-specific probes, or at the DNA level following reverse transcription (and cloning) of the RNA population, for example by quantitative PCR or real-time PCR techniques. The term "determining", as used herein, includes the analysis of any nucleic acid molecules encoding a microRNA sequence as described above. However, due to the short half-life of pri-miRNAs and pre-mRNAs typically the concentration of only the mature miRNA is measured.

In specific embodiments, the standard value of the expression levels obtained in several independent measurements of a given sample (for example, two, three, five or ten measurements) and/or several measurements within a population of cancer stem cells (target cells) or control cells is used for analysis. The standard value may be obtained by any method known in the art. For example, a range of mean ± 2 SD (standard deviation) or mean ± 3 SD may be used as standard value.

The difference between the expression levels obtained for one or more target cells and one or more control cells may also be normalized to the expression level of further control nucleic acids, e.g. U6 RNA or housekeeping genes whose expression levels are known not to differ depending on the disease states of the cell. Exemplary housekeeping genes include *inter alia* β-actin, glycerinaldehyde 3-phosphate dehydrogenase, and ribosomal protein P1. Preferably, the control nucleic acid for normalizing the expression levels obtained is another miRNA known to be stably expressed during the various non-cancerous and cancerous (or precancerous) states of the cell.

However, instead of determining in any experiment the expression levels for one or more control cells it may also be possible to define, based on experimental evidence and/or prior art data, one or more cut-off values for a particular cell phenotype (i.e. a cancerous state, a precancerous condition (i.e., as used herein, a state of disposition to develop a cancerous state) or a control state). In such scenario, the respective expression levels for the one or more target cells can be determined by using a stably expressed control miRNA for normalization. If the "normalized" expression levels calculated are higher than the respective cutoff value defined, then this finding is be indicative for an up-regulation of gene expression. *Vice versa,* if the "normalized" expression levels calculated are lower than the respective cutoff value defined, then this finding would be indicative for a down-regulation of gene expression.

In the context of the present invention, the term "identifying one or more cancer stem cells" is intended to also encompass predictions and likelihood analysis (in the sense of "diagnosing"). The compositions and methods disclosed herein are intended to be used clinically in making decisions with regard to treatment modalities, including therapeutic intervention, diagnostic criteria such as disease stages, and disease monitoring and surveillance for the cancer in question. According to the present invention, an intermediate result for examining the condition of a subject may be provided. Such intermediate result may be combined with additional information to assist a doctor, nurse, or other practitioner to diagnose that a subject suffers from a particular cancer.

Within the present invention, one or more differentially expressed nucleic acid molecules identified together represent a nucleic acid expression signature that is indicative for the presence of cancer stem cells. The term "expression signature", as used herein, denotes a set of nucleic acid molecules (e.g., miRNAs), wherein the expression level of the individual nucleic acid molecules differs between the cancer stem cells and the control cells. Herein, a nucleic acid expression signature is also referred to as a set of markers and represents a minimum number of (different) nucleic acid molecules each encoding a miRNA sequence that is capable for identifying a phenotypic state of a target cell. In other words, according to the present invention the expression signature in its entirety (i.e. the one or more differentially expressed nucleic acid molecules together) is indicative for the presence of cancer stem cells but not the mere differential expression of an individual nucleic acid molecule.

In specific embodiments, the nucleic acid expression signature comprises at least three nucleic acid molecules, each encoding a (different) miRNA sequence. Preferably, the nucleic acid expression signature comprises at least five or more preferably at least eight (different) nucleic acid molecules. In other specific embodiments, the nucleic acid signature comprises at least ten or at least twelve (different) nucleic acid molecules.

Typically, the nucleic acid molecules comprised in the nucleic acid expression signature are human sequences (hereinafter designated "hsa" for "Homo sapiens").

In particular preferred embodiments of the invention, the nucleic acid expression signature of the diagnostic kit comprises nucleic acid molecules encoding microRNA sequences selected from the group consisting of hsa-miR-9 (SEQ ID NO:1) and hsa-miR-9* (SEQ ID NO:5).

It is to be understood that the expression signature comprises nucleic acid molecules encoding hsa-miR-9 and nucleic acid molecules encoding hsa-miR-9*. In some embodiments, however, it is also possible that the expression signature only comprises nucleic acid molecules encoding hsa-miR-9 or only comprises nucleic acid molecules encoding hsa-miR-9*.

Both hsa-miR-9 and hsa-miR-9* were reported to represent brain-specific/neuronal miRNAs and to be expressed specifically in the developing nervous system (Kapsimali, M. et al. (2007) Genome Biol. 8, R173; Landgraf, P. et al. (2007) Cell 129, 1401-1414). Contradictory data, however, appear to exist with respect to a potential role these miRNAs may exert during brain tumor progression: both in neuroblastoma and medullolastoma a down-regulation of hsa-miR-9/hsa-miR-9* was observed (Laneve, P. et al. (2008) Proc. Natl. Acad. Sci. USA 104, 7957-7962; Ferretti, E. et al. (2009) Int. J. Cancer 124, 568-577, published online on 30 October 2008), while a third study reported an up-regulation in primary brain tumors (Nass, D. et al. (2009) Brain Pathol. ISSN 1015-6305, published online on 02 July 2008).

In preferred embodiments of the present invention, the expression of any of the nucleic acid molecules encoding hsa-miR-9 and hsa-miR-9* is up-regulated in the one or more cancer stem cells compared to the one or more control cells.

Three miRNA precursors are known for either of the two above-references miRNAs. The respective precursors of hsa-miR-9 are given in SEQ ID NO:2, SEQ ID NO:3, and SEQ ID NO:4, whereas respective precursors of hsa-miR-9* are given in SEQ ID NO:6, SEQ ID NO:7, and SEQ ID NO:8.

All mature miRNA sequences as well as their corresponding precursors disclosed herein have been deposited in the miRBase database (http://microrna.sanger.ac.uk/; see also Griffiths-Jones S. et al. (2008) Nucl. Acids Res. 36, D154-D158).

In further specific embodiments, the nucleic acid expression signature comprises at least one nucleic acid molecule encoding a miRNA sequence whose expression is up-regulated (i.e. its concentration is increased) in the one or more cancer stem cells compared to the one or more control cells and at least one nucleic acid molecule encoding a miRNA sequence whose expression is down-regulated (i.e. its concentration is reduced) in the one or more cancer stem cells compared to the one or more control cells.

In other specific embodiments, the nucleic acid expression signature further (i.e. in addition to nucleic acid molecules encoding hsa-miR-9 and hsa-miR-9*) comprises any one or more nucleic acid molecules encoding microRNA sequences selected from the group consisting of hsa-miR-17-5p (SEQ ID NO:9), hsa-miR-106b (SEQ ID NO:11), hsa-miR-15b (SEQ ID NO:13), hsa-miR-151-5p (SEQ ID NO:15), hsa-miR-320 (SEQ ID NO: 17), hsa-miR-23b (SEQ ID NO:19), hsa-miR-25 (SEQ ID NO:21), hsa-miR-191 (SEQ ID NO:23), hsa-miR-15a (SEQ ID NO:25), hsa-miR-103 (SEQ ID NO:27), hsa-miR-16 (SEQ ID NO:30), hsa-miR-221 (SEQ ID NO:33), hsa-miR-222 (SEQ ID NO:35), hsa-miR-27a (SEQ ID NO:37), hsa-miR-21 (SEQ ID NO:39), hsa-miR-26a (SEQ ID NO:41), hsa-miR-23a (SEQ ID NO:44), and hsa-miR-27b (SEQ ID NO:46).

The respective precursors of the above-referenced miRNAs are given in SEQ ID NO:10 (hsa-miR-17-5p), SEQ ID NO:12 (hsa-miR-106b), SEQ ID NO:14 (hsa-miR-15b), SEQ ID NO:16 (hsa-miR-151-5p), SEQ ID NO:18 (hsa-miR-320), SEQ ID NO:20 (hsa-miR-23b), SEQ ID NO:22 (hsa-miR-25), SEQ ID NO:24 (hsa-miR-191), SEQ ID NO:26 (hsa-miR-15a), SEQ ID NO:28 and SEQ ID NO:29 (two precursors for hsa-miR-103),), SEQ ID NO:31 and SEQ ID NO:32 (two precursors for hsa-miR-16), SEQ ID NO:34 (hsa-miR-221), SEQ ID NO:36 (hsa-miR-222), SEQ ID NO:38 (hsa-miR-27a), SEQ ID NO:40 (hsa-miR-21), ), SEQ ID NO:42 and SEQ ID NO:43 (two precursors for hsa-miR-26a), SEQ ID NO:45 (hsa-miR-23a), and SEQ ID NO:47 (hsa-miR-27b).

Preferably, the expression of any of the nucleic acid molecules encoding hsa-miR-17-5p, hsa-miR-106b, hsa-miR-15b, hsa-miR-151-5p, hsa-miR-320, hsa-miR-23b, hsa-miR-25, hsa-miR-191, hsa-miR-15a, hsa-miR-103, and hsa-miR-16 is up-regulated and the expression of any of the nucleic acid molecules hsa-miR-221, hsa-miR-222, hsa-miR-27a, hsa-miR-21, hsa-miR-26a, hsa-miR-23a, and hsa-miR-27b is down-regulated in the in the one or more target cells compared to the one or more control cells.

The terms "one or more of the plurality of nucleic acid molecules" and "any one or more human target cell-derived nucleic acid molecules", as used herein, may relate to any subgroup of the plurality of nucleic acid molecules, e.g., any one, any two, any three, any four, any five, any six, any seven, any eight, any nine, any ten, and so forth nucleic acid molecules, each encoding a microRNA sequence that are comprised in the nucleic acid expression signature, as defined herein. Thus, in other words, the nucleic acid expression signature includes at least nucleic acid molecules encoding hsa-miR-9 and hsa-miR-9* but may contain one or more additional nucleic acid molecules encoding any further miRNA sequences that are differentially expressed in the target cells and in one or more control cells analyzed, particularly one or more additional nucleic acid molecules encoding any one of the remaining miRNA sequences referred to above (i.e., hsa-miR-17-5p, hsa-miR-106b, hsa-miR-15b, hsa-miR-151-5p, hsa-miR-320, hsa-miR-23b, hsa-miR-25, hsa-miR-191, hsa-miR-15a, hsa-miR-103, hsa-miR-16, hsa-miR-221, hsa-miR-222, hsa-miR-27a, hsa-miR-21, hsa-miR-26a, hsa-miR-23a, and hsa-miR-27b).

In particularly preferred embodiments, the nucleic acid expression signature comprises nucleic acid molecules encoding hsa-miR-9, hsa-miR-9*, hsa-miR-17-5p, hsa-miR-106b, hsa-miR15b, hsa-miR-221, hsa-miR-222, hsa-miR-27a, and hsa-miR-21.

In further particularly preferred embodiments, the nucleic acid expression signature comprises nucleic acid molecules encoding hsa-miR-9, hsa-miR-9*, hsa-miR-17-5p, hsa-miR-106b, hsa-miR-15b, hsa-miR-151-5p, hsa-miR-320, hsa-miR-23b, hsa-miR-25, hsa-miR-191, hsa-miR-15a, hsa-miR-103, hsa-miR-16, hsa-miR-221, hsa-miR-222, hsa-miR-27a, hsa-miR-21, hsa-miR-26a, hsa-miR-23a, and hsa-miR-27b.

The nucleic acid sequences of the mature miRNAs disclosed herein are listed in Table 1, the nucleic acid sequences of the corresponding miRNA precursors in Table 2.

In a second aspect, the present invention relates to a method for identifying one or more cancer stem cells, the method comprising:
(a) determining in the one or more cancer stem cells the expression levels of a plurality of nucleic acid molecules, each nucleic acid molecule encoding a microRNA sequence;
(b) determining the respective expression levels of the plurality of nucleic acid molecules in one or more control cells; and
(c) identifying from the plurality of nucleic acid molecules one or more nucleic acid molecules that are differentially expressed in the target and control cells by comparing the respective expression levels obtained in steps (a) and (b),
wherein the one or more differentially expressed nucleic acid molecules together represent a nucleic acid expression signature, as defined herein, that is indicative for the presence of cancer stem cells. Preferably, the cancer stem cells to be analyzed are CD133-positive.

In preferred embodiments, the method is performed as an *in vitro* method.

The method of the present invention comprises determining and comparing the expression levels of a plurality of nucleic acid molecules encoding a microRNA sequence both in one or more cancer stem cells and in one or more control cells (cf. also the discussion above). In preferred embodiments, the method is performed as an *in vitro* method.

In other preferred embodiments, the cancer stem cells are CD133-positive and the control cells are CD133-negative cancer cells, and the method further comprises separating the CD133-positive and CD133-negative cells prior to performing step (a).

Separation of CD133-positive and CD133-negative cell fractions comprised in a sample may preferably be accomplished by means of fluorescent labeling CD133⁺ cells (e.g. with an anti-CD133-phycoerythrin antibody), fluorescence-assisted cell sorting (FACS), and separation of cell fractions by flow cytometry. Alternatively, separation of CD133-positive cells may be accomplished by magnetic labeling of CD133⁺ cells (e.g., with a magnetic anti-CD133 antibody) and subsequent magnetic-assisted cell sorting (MACS). All these techniques are well established in the art.

In a third aspect, the invention relates to a method for preventing the proliferation and/or self-renewal of one or more cancer stem cells, the method comprising:
(a) identifying in the one or more cancer stem cells a nucleic acid expression signature by using a method, as defined herein; and
(b) modifying in the one or more cancer stem cells the expression of one or more nucleic acid molecules encoding a microRNA sequence that is/are comprised in the nucleic acid expression signature in such way that the expression of a nucleic acid molecule whose expression is up-regulated in the one or more cancer stem cells is down-regulated and the expression of a nucleic acid molecule whose expression is down-regulated in the one or more cancer stem cells is up-regulated.

In preferred embodiments, the cancer stem cells to be analyzed are CD133-positive. The control cells employed are preferably CD133-negative cancer cells.

In further preferred embodiments, the method is performed as an *in vitro* method.

The term "preventing the proliferation and/or self-renewal", as used herein, refers to any form of interference with cancer stem cell growth and cell division, particularly an inhibition of the ability of cancer stem cells to self-renew that triggers massive proliferation and maintenance of a tissue-specific undifferentiated pool of cells in an organ or tissue, and thus tumor progression and/or regeneration. Within the present invention, the term also includes the promotion of cancer stem cell differentiation, that is, the production of daughter cells that become tissue-specific specialized cells. As used herein, the term also includes the induction of cell death of cancer stem cells. Hence, the method according to the present invention aims at a reduction or complete elimination of the fraction of cancer stem cells in a given population of tumor cells or a tumor tissue.

The term "modifying the expression of a nucleic acid molecule encoding a miRNA sequence", as used herein, denotes any manipulation of a particular nucleic acid molecule resulting in an altered expression level of said molecule, that is, the production of a different amount of corresponding miRNA as compared to the expression of the "wild-type" (i.e. the unmodified control). The term "different amount", as used herein, includes both a higher amount and a lower amount than determined in the unmodified control. In other words, a manipulation, as defined herein, may either up-regulate (i.e. activate) or down-regulate (i.e. inhibit) the expression (i.e. particularly transcription) of a nucleic acid molecule.

Within the present invention, expression of one or more nucleic acid molecules encoding a microRNA sequence comprised in the nucleic acid expression signature is modified in such way that the expression of a nucleic acid molecule whose expression is up-regulated in the one or more target cells is down-regulated and the expression of a nucleic acid molecule whose expression is down-regulated in the one or more target cells is up-regulated. In other words, the modification of expression of a particular nucleic acid molecule encoding a miRNA sequence occurs in an anti-cyclical pattern to the regulation of said molecule in the one or more cancerous target cells in order to interfere with the "excess activity" of an up-regulated molecule and/or to restore the "deficient activity" of a down-regulated molecule in the one or more target cells.

In a preferred embodiment of the inventive method, down-regulating the expression of a nucleic acid molecule comprises introducing into the one or more target cells a nucleic acid molecule encoding a sequence that is complementary to the microRNA sequence encoded by nucleic acid molecule to be down-regulated.

The term "introducing into a cell", as used herein, refers to any manipulation allowing the transfer of one or more nucleic acid molecules into a cell. Examples of such techniques include *inter alia* transfection or transduction techniques or the delivery of chemically modified nucleic acids (e.g., by coupling to cholesterol). All these methods are well established in the art (cf., for example, Sambrook, J. et al. (1989) Molecular, Cloning: A Laboratory Manual, 2nd ed., Cold Spring Harbor Laboratory Press, Cold Spring Harbor, NY; Ausubel, F.M. et al. (2001) Current Protocols in Molecular Biology, Wiley & Sons, Hoboken, NJ).

The term "complementary sequence", as used herein, is to be understood that the "complementary" nucleic acid molecule (herein also referred to as an "anti-sense nucleic acid molecule") introduced into the one or more cells is capable of forming base pairs, preferably Watson-Crick base pairs, with the up-regulated endogenous "sense" nucleic acid molecule.

Two nucleic acid molecules (i.e. the "sense" and the "anti-sense" molecule) may be perfectly complementary, that is, they do not contain any base mismatches and/or additional or missing nucleotides. In other embodiments, the two molecules comprise one or more base mismatches or differ in their total numbers of nucleotides (due to additions or deletions). In further embodiments, the "complementary" nucleic acid molecule comprises at least ten contiguous nucleotides showing perfect complementarity with a sequence comprised in the up-regulated "sense" nucleic acid molecule.

The "complementary" nucleic acid molecule (i.e. the nucleic acid molecule encoding a nucleic acid sequence that is complementary to the microRNA sequence encoded by nucleic acid molecule to be down-regulated) may be a naturally occurring DNA- or RNA molecule or a synthetic nucleic acid molecule comprising in its sequence one or more modified nucleotides which may be of the same type or of one or more different types.

For example, it may be possible that such a nucleic acid molecule comprises at least one ribonucleotide backbone unit and at least one deoxyribonucleotide backbone unit. Furthermore, the nucleic acid molecule may contain one or more modifications of the RNA backbone into 2'-*O*-methyl group or 2'-*O*-methoxyethyl group (also referred to as "2'-*O-*methylation"), which prevented nuclease degradation in the culture media and, importantly, also prevented endonucleolytic cleavage by the RNA-induced silencing complex nuclease, leading to irreversible inhibition of the miRNA. Another possible modification, which is functionally equivalent to 2'-*O*-methylation, involves locked nucleic acids (LNAs) representing nucleic acid analogs containing one or more LNA nucleotide monomers with a bicyclic furanose unit locked in an RNA-mimicking sugar conformation (cf., e.g., Orom, U.A. et al. (2006) Gene 372, 137-141).

Another class of silencers of miRNA expression was recently developed. These chemically engineered oligonucleotides, named "antagomirs", represent single-stranded 23-nucleotide RNA molecules conjugated to cholesterol (Krutzfeldt, J. et al. (2005) Nature 438, 685-689). As an alternative to such chemically modified oligonucleotides, microRNA inhibitors that can be expressed in cells, as RNAs produced from transgenes, were generated as well. Termed "microRNA sponges", these competitive inhibitors are transcripts expressed from strong promoters, containing multiple, tandem binding sites to a microRNA of interest (Ebert, M.S. et al. (2007) Nat. Methods 4, 721-726).

In preferred embodiments of the inventive method, the one or more nucleic acid molecules whose expression is to be down-regulated encode microRNA sequences selected from the group consisting of hsa-miR-9, hsa-miR-9*, hsa-miR-17-5p, hsa-miR-106b, hsa-miR-15b, hsa-miR-151-5p, hsa-miR-320, hsa-miR-23b, hsa-miR-25, hsa-miR-191, hsa-miR-15a, hsa-miR-103, and hsa-miR-16, with hsa-miR-9 and hsa-miR-9* being particularly preferred.

In a further preferred embodiment of the inventive method, up-regulating the expression of a nucleic acid molecule comprises introducing into the one or more target cells a nucleic acid molecule encoding the microRNA sequence encoded by nucleic acid molecule to be up-regulated. In other words, the up-regulation of the expression of a nucleic acid molecule encoding a miRNA sequence is accomplished by introducing into the one or more cells another copy of said miRNA sequence (i.e. an additional "sense" nucleic acid molecule). The "sense" nucleic acid molecule to be introduced into the one or more target cells may comprise the same modification as the "anti-sense" nucleic acid molecules described above.

In further preferred embodiments of the inventive method, the one or more nucleic acid molecules whose expression is to be up-regulated encode microRNA sequences selected from the group consisting of hsa-miR-221, hsa-miR-222, hsa-miR-27a, hsa-miR-21, hsa-miR-26a, hsa-miR-23a, and hsa-miR-27b.

The "sense" and/or the "anti-sense" nucleic acid molecules to be introduced into the one ore more target cells in order to modify the expression of one or more nucleic acid molecules encoding a microRNA sequence that is/are comprised in the nucleic acid expression signature may be operably linked to a regulatory sequence in order to allow expression of the nucleotide sequence.

In order to unravel any potential implication of the miRNAs identified in the cancer stem cells functional analyses may be performed with respect to the identification of mRNA target sequences to which the miRNAs may bind. Based on the finding that miRNAs may be involved in both tumor suppression and tumorigenesis (reviewed, e.g., in Esquela-Kerscher, A. and Slack, F.J (2006) *supra*; Calin, G.A. and Croce, C.M. (2007) *supra*; Blenkiron, C. and Miska, E.A. (2007) *supra*) it is likely to speculate that mRNA target sites for such miRNAs include tumor suppressor genes as well as oncogenes.

And indeed, in preliminary analyses it was shown that inhibition of hsa-miR-9* expression resulted in the overall down-regulation of the Wnt/β-catenin pathway, which is associated with many types of cancer (e.g., brain tumors, melanoma, breast cancer, colorectal cancer, leukemia) and also implicated in cell maintenance/self-renewal and regenerative responses during tissue repair (reviewed, e.g., in Taipale, J. and Beachy, P.A. (2001) Nature 411, 349-354). Exemplary Wnt/β-catenin pathway-associated potential target genes that were down-regulated after inhibition of hsa-miR-9* expression include: WISP1 gene (Wnt1 inducible signaling pathway protein 1), EN1 gene (engrailed homeobox 1), v-myc, TWSG1 gene (twisted gastrulation homolog 1), KLF4 gene (Krüppel-like factor 4), L1CAM gene (L1 cell adhesion molecule), VEGFA gene (vascular endothelial growth factor A), BCL9L gene (B-cell CLL/lymphoma 9-like factor), and CDX1 gene (Caudal type homeobox). Further potential target genes down-regulated after hsa-miR-9* inhibition include, for example, multiple histone genes, the GATA4 gene (encoding the GATA binding protein 4 that is overexpressed in many cancers) the GBX2 gene (encoding the gastrulation brain homeobox 2 protein, which stimulates cell proliferation in many cancers), and the RFC3 gene (encoding the replication factor C activator 1). These results demonstrate that inhibition of hsa-miR-9* expression in fact appears to impair proliferation and self-renewal of cancer stem cells.

A nucleic acid molecule is referred to as "capable of expressing a nucleic acid molecule" or capable "to allow expression of a nucleotide sequence" if it comprises sequence elements which contain information regarding to transcriptional and/or translational regulation, and such sequences are "operably linked" to the nucleotide sequence encoding the polypeptide. An operable linkage is a linkage in which the regulatory sequence elements and the sequence to be expressed (and/or the sequences to be expressed among each other) are connected in a way that enables gene expression.

The precise nature of the regulatory regions necessary for gene expression may vary among species, but in general these regions comprise a promoter which, in prokaryotes, contains both the promoter *per se,* i.e. DNA elements directing the initiation of transcription, as well as DNA elements which, when transcribed into RNA, will signal the initiation of translation. Such promoter regions normally include 5' non-coding sequences involved in initiation of transcription and translation, such as the -35/-10 boxes and the Shine-Dalgarno element in prokaryotes or the TATA box, CAAT sequences, and 5'-capping elements in eukaryotes. These regions can also include enhancer or repressor elements as well as translated signal and leader sequences for targeting the native polypeptide to a specific compartment of a host cell.

In addition, the 3' non-coding sequences may contain regulatory elements involved in transcriptional termination, polyadenylation or the like. If, however, these termination sequences are not satisfactory functional in a particular host cell, then they may be substituted with signals functional in that cell.

Furthermore, the expression of the nucleic molecules, as defined herein, may also be influenced by the presence, e.g., of modified nucleotides (cf. the discussion above). For example, locked nucleic acid (LNA) monomers are thought to increase the functional half-life of miRNAs *in vivo* by enhancing the resistance to degradation and by stabilizing the miRNA-target duplex structure that is crucial for silencing activity (cf., e.g., Naguibneva, I. et al. (2006) Biomed. Pharmacother. 60, 633-638).

Therefore, a nucleic acid molecule of the invention to be introduced into the one or more cells provided may include a regulatory sequence, preferably a promoter sequence, and optionally also a transcriptional termination sequence.

The promoters may allow for either a constitutive or an inducible gene expression. Suitable promoters include *inter alia* the *E. coli lacUV5* and *tet* (tetracycline-responsive) promoters, the T7 promoter as well as the SV40 promoter or the CMV promoter.

The nucleic acid molecules of the invention may also be comprised in a vector or other cloning vehicles, such as plasmids, phagemids, phages, cosmids or artificial chromosomes. In a preferred embodiment, the nucleic acid molecule is comprised in a vector, particularly in an expression vector. Such an expression vector can include, aside from the regulatory sequences described above and a nucleic acid sequence encoding a genetic construct as defined in the invention, replication and control sequences derived from a species compatible with the host that is used for expression as well as selection markers conferring a selectable phenotype on transfected cells. Large numbers of suitable vectors such as pSUPER and pSUPERIOR are known in the art, and are commercially available.

In a forth aspect, the present invention relates to a pharmaceutical composition for preventing the proliferation and/or self-renewal of one or more cancer stem cells, the composition comprising one or more nucleic acid molecules, each nucleic acid molecule encoding a sequence that is at least partially complementary to a microRNA sequence encoded by a nucleic acid molecule whose expression is up-regulated in the one or more cancer stem cells, as defined herein, and/or that corresponds to a microRNA sequence encoded by a nucleic acid molecule whose expression is down-regulated in the one or more cancer stem cells, as defined herein. Preferably, the cancer stem cells are CD133-positive.

In further preferred embodiments, the pharmaceutical composition is for preventing the proliferation and/or self-renewal of cancer stem cells derived from neuronal and/or glial tumors, particularly preferably from gliobastoma.

Finally, the present invention relates to the use of said pharmaceutical composition for the manufacture of a medicament for the prevention and/or treatment of cancer, preferably of neuronal and/or glial cancers, particularly preferably for glioblastoma.

In the context of the present invention, suitable pharmaceutical compositions include those suitable for oral, rectal, nasal, topical (including buccal and sub-lingual), peritoneal and parenteral (including intramuscular, subcutaneous and intravenous) administration, or for administration by inhalation or insufflation. Administration may be local or systemic. Preferably, administration is accomplished via the oral, rectal or intravenous routes. The formulations may be packaged in discrete dosage units.

Pharmaceutical compositions according to the present invention include any pharmaceutical dosage forms established in the art, such as *inter alia* capsules, microcapsules, cachets, pills, tablets, powders, pellets, multi-particulate formulations (e.g., beads, granules or crystals), aerosols, sprays, foams, solutions, dispersions, tinctures, syrups, elixirs, suspensions, water-in-oil emulsions such as ointments, and oil-in water emulsions such as creams, lotions, and balms.

The ("sense" and "anti-sense") nucleic acid molecules described above can be formulated into pharmaceutical compositions using pharmacologically acceptable ingredients as well as established methods of preparation (Gennaro, A.L. and Gennaro, A.R. (2000) Remington: The Science and Practice of Pharmacy, 20th Ed., Lippincott Williams & Wilkins, Philadelphia, PA; Crowder, T.M. et al. (2003) A Guide to Pharmaceutical Particulate Science. Interpharm/CRC, Boca Raton, FL; Niazi, S.K. (2004) Handbook of Pharmaceutical Manufacturing Formulations, CRC Press, Boca Raton, FL).

In order to prepare the pharmaceutical compositions, pharmaceutically inert inorganic or organic excipients (i.e. carriers) can be used. To prepare e.g. pills, tablets, capsules or granules, for example, lactose, talc, stearic acid and its salts, fats, waxes, solid or liquid polyols, natural and hardened oils may be used. Suitable excipients for the production of solutions, suspensions, emulsions, aerosol mixtures or powders for reconstitution into solutions or aerosol mixtures prior to use include water, alcohols, glycerol, polyols, and suitable mixtures thereof as well as vegetable oils. The pharmaceutical composition may also contain additives, such as, for example, fillers, binders, wetting agents, glidants, stabilizers, preservatives, emulsifiers, and furthermore solvents or solubilizers or agents for achieving a depot effect. The latter is to be understood that the nucleic acid molecules may be incorporated into slow or sustained release or targeted delivery systems, such as liposomes, nanoparticles, and microcapsules.

To target most tissues within the body, clinically feasible noninvasive strategies are required for directing such pharmaceutical compositions, as defined herein, into cells. In the past years, several approaches have achieved impressive therapeutic benefit following intravenous injection into mice and primates using reasonable doses of siRNAs without apparent limiting toxicities.

One approach involves covalently coupling the passenger strand (miRNA* strand) of the miRNA to cholesterol or derivatives/conjugates thereof to facilitate uptake through ubiquitously expressed cell-surface LDL receptors (Soutschek, J. et al. (2004) Nature 432, 173-178). Alternatively, unconjugated, PBS-formulated locked-nucleic-acid-modified oligonucleotides (LNA-antimiR) may be used for systemic delivery (Elmen, J. et al. (2008) Nature 452, 896-899). Another strategy for delivering miRNAs involves encapsulating the miRNAs into specialized liposomes formed using polyethylene glycol to reduce uptake by scavenger cells and enhance time spent in the circulation. These specialized nucleic acid particles (stable nucleic acid-lipid particles or SNALPs) delivered miRNAs effectively to the liver (and not to other organs (cf., e.g., Zimmermann, T.S. et al. (2006) Nature 441, 111-114). Recently, a new class of lipid-like delivery molecules, termed lipidoids (synthesis scheme based upon the conjugate addition of alkylacrylates or alkyl-acrylamides to primary or secondary amines) has been described as delivery agents for RNAi therapeutics (Akinc, A. et al. (2008) Nat. Biotechnol. 26, 561-569).

A further cell-specific targeting strategy involves the mixing of miRNAs with a fusion protein composed of a targeting antibody fragment linked to protamine, the basic protein that nucleates DNA in sperm and binds miRNAs by charge (Song, E. et al. (2005) Nat. Biotechnol. 23, 709-717). Multiple modifications or variations of the above basic delivery approaches have recently been developed. These techniques are known in the art and reviewed, e.g., in de Fougerolles, A. et al. (2007) Nat. Rev. Drug Discov. 6, 443-453; Kim, D.H. and Rossi, J.J. (2007) Nat. Genet. 8, 173-184).

The invention is further described by the figures and the following examples, which are solely for the purpose of illustrating specific embodiments of this invention, and are not to be construed as limiting the scope of the invention in any way.

### EXAMPLES

### Example 1: Materials and Methods

### 1.1. Cell Culture

The generation of R11, R20, R28, R40, R44, and R52 cell lines from glioblastoma samples was performed as previously described (Beier, D. et al. (2007) Cancer Res. 67, 4010-4015).

### 1.2. Antibodies

The following antibodies were used: mouse anti-Tuj1 MMS-435p (from Covance), mouse anti-β-actin AC15 (from Abcam), rabbit anti-GFAP (from DAKO), anti-CD133-2 293C3-PE (from Miltenyi), anti-rabbit-HRP, anti-mouse-HRP (both from Sigma).

### 1.3. Oligonucleotides

2'O methyl oligonucleotides were chemically synthesized using RNA phosphoramidites (from Pierce) on a Äkta Oligopilot 10 DNA/RNA synthesizer (from GE healthcare), according to the manufacturer's protocol. The sequences were as follows:

| | |
|---|---|
| hsa-miR-9* antisense: | 5'-ACUUUCGGUUAUCUAGCUUUAdT |
| hsa-miR-17-5p antisense: | 5'-ACUACCUGCACUGUAAGCACUUUGdT |
| hsa-miR-106b antisense: | 5'-AUCUGCACUGUCAGCACUUUAdT |
| hsa-miR-9 antisense: | 5'-UCAUACAGCUAGAUAACCAAAGAdT |
| hsa-miR-122 antisense: | 5'-ACAAACACCAUUGUCACACUCCAdT |
| Ile tRNA probe: | 5'-TGCTCCAGGTGAGGATCGAAC |

### 1.4. Plasmids

The pMIR-RL dual luciferase vector was previously described (Beitzinger, M., et al. (2007) RNA Biol. 4, 76-84). 3'-UTRs of candidate miRNA target mRNAs were amplified via PCR from R28 genomic DNA using gene-specific primers. For Onecut2, the following 3'-UTR fragments were amplified: fragment 1 (nucleotides 5211-10140) and fragment 2 (nucleotides 10141-14574). PCR products were digested with appropriate restriction enzymes and ligated into pMIR-RL.

### 1.5. Cell culture and transfection

R11, R20, R28, R40, R44, and R52 cell lines were cultured at 37°C in atmosphere containing 5% CO₂ in DMEM-F12 medium supplemented with 20 ng/ml each of human recombinant epidermal growth factor, human recombinant basic fibroblast growth factor (both from R&D Systems), and human leukemia inhibitory factor (from Millipore), 2% B27 supplement, 1% MEM vitamins solution (both from Invitrogen), and 1% penicillin/streptomycin solution (from PAA). Cells were passaged every 10-14 days by trypsinization or by detaching with a pipette. 50% of the medium was substituted twice weekly. T98G cells were cultured in DMEM supplemented with 10% fetal bovine serum and 1% penicillin/streptomycin solution (both from PAA). Typically, cells were passaged every 3 days by trypsinization in a 1:10 ratio.

R11 cells were reverse transfected with 2'O methyl oligonucleotides in 6 well plates with 5 µl/well Lipofectamine 2000 (from Invitrogen). The transfection mix was removed 24 hrs post transfection, and fresh medium was added. T98G cells were transfected with Lipofectamine 2000 12 hrs after seeding according to the manufacturer's instructions.

### 1.6. Fluorescence-assisted cell sorting (FACS)/Flow cytometry

Cells were trypsinized, washed with DMEM-F12 and FACS buffer (PBS containing 1% (w/v) BSA), resuspended in 500 µl FACS buffer containing 10% FcR blocking reagent (from Miltenyi) and incubated for 5 min on ice. 5 µl anti-CD133-PE was added, and cells were incubated for 10 min on ice in the dark. Cells were pelleted and washed once with FACS buffer. Stained cells were sorted on a FACS Aria system (from Becton Dickinson). Cell debris was gated out using a forward scatter/sideward scatter dot plot. CD133-negative and CD133-positive cell populations were identified using unstained cells as control.

### 1.7. RNA isolation

Total RNA for mRNA analyses was isolated using the Prep Ease kit (form USB), according to the manufacturer's instructions. For small RNA detection, RNA was isolated using Trifast (from Peqlab), also following the manufacturer's protocol.

### 1.8. cDNA synthesis

cDNA for mRNA analysis was synthesized with random hexamer primers from 2 µg of total RNA using the First Strand cDNA synthesis kit (from Fermentas), according to the manufacturer's protocol. In order to quantify miRNAs, RNA samples were treated with DNaseI (also from Fermentas), poly(A)-tailed using the poly(A) tailing kit (from Ambion) and reverse transcribed using the First Strand cDNA synthesis kit and the URT primer 5'-AACGAGACGACGACAGACTTTTTTTTTTTTTTTV-3' (Hurteau, G. J. et al. (2006) Cell Cycle 5, 1951-1956).

### 1.9. Microarray experiments

Total RNA samples from R11 cells transfected with 2'O methyl antisense oligonucleotides for hsa-miR-122 (control) or hsa-miR-9* (see above) were processed for array hybridization by Miltenyi Biotec. In brief, RNA quality was assessed on an Agilent 2100 Bioanalyzer, and SuperAmp RNA amplification was carried out. Corresponding cDNA samples were labeled with Cy3 and hybridized to Agilent human 4x44k whole genome microarrays. RNA expression values were normalized to the 50th percentile of the median of each chip, and genes, whose expression was down-regulated (repressed) or up-regulated (increased) by a factor of > 2, were identified.

### 1.10. Quantitative PCR (qPCR)

qPCR was performed on a MyiQ cycler (from BioRad) using the Mesa Green qPCR Mastermix (from Eurogenetec). The sequences of the primers employed were as follows: GAPDH, 5'-TGGTATCGTGGAAGGACTCATGAC-3', 5'-ATGCCAGTGAGCTTCCCGTTCAGC-3'; β-Actin, 5'-CTGGAGAAGAGCTACGAGCTG-3', 5'-TTGAAGGTAGTTTCGTGGATG-3'; hsa-miR-9, 5'-TCTTTGGTTATCTAGCTGTATG-3'; hsa-miR-9*, 5'-ATAAAGCTAGATAA CCGAAAG-3'; U6 snRNA, 5'-GATGACACGCAAATTCGTGAAG-3'; universal RT primer for miRNA and U6 snRNA detection, 5'-AACGAGACGACGACAGACTTT-3'; Onecut1, 5'-CAAAAGGAAAGAACAAGAACATG-3', 5'-TATTGCATGTAGAGTTCGAC-3'; Onecut2, 5'-GCCACGACAAAATGCTCAGC-3', 5'-CGTTCAGGTGCGACATCATGG-3'; WISP1, 5'-CGATGTGGACATCCATACACTC-3', 5'-GCAAGTGTGAAGTTCATGGATG-3'; HIST1 HIE, 5'-GAGCTCATTACTAAAGCTGTTG-3', 5'-TTGTTCTTCTCCACGTCATAGC-3'; HIST1H2AJ, 5'-CTGCCAAAGAAAACTGAGAGC-3', 5'-GAAAAGAGCCTTTGTTTTTA TGC-3'; HIST1H2BJ, 5'-GCATCATGAATTCGTTTGTGAAC-3', 5'-CCTGGAGGTGATG GTCGAGC-3'; HIST1H3D, 5'-CAACGACGAGGAGCTAAACAAG-3', 5'-TGGTGACTCT CAGTCTTCTTG-3'; HIST1H4G, 5'-GCATTACCAAGTGCACTATC-3', 5'-TTTCCAGGA ACACCTTGAACAC-3'; HIST2H2AC, 5'-CAACGACGAGGAACTGAACAAG-3', 5'-GTT TTCTTTGGTAACAGAACG-3'; HIST3H2BB, 5'-CCTGGCACACTACAACAAGC-3', 5'-GAGCTGGTGTACTTGGTGACAG-3'. Data were evaluated using the ddCt method, using GAPDH or β-actin as reference mRNAs. Error bars were obtained from triplicate PCR samples by propagating the ddCt standard error of the mean through the exponential term as previously described (Livak, K.J. and Schmittgen, T.D. (2001) Meth. Methods 25, 402-408).

### 1.11. Generation and sequencing of small RNA libraries

Small RNA libraries were generated by Vertis Biotechnology AG (Freising-Weihenstephan, Germany) and sequenced by 454 pyrosequencing as previously described (Tarasov, V. et al. (2007) Cell Cycle 6, 1586-1593).

### 1.12. Analysis of sequencing results

Known miRNAs were identified by means of comparing the sequencing results with annotated miRNAs from the *H. sapiens* miRNA database (miRBase; Criffiths-Jones, S. (2008), *supra*) using the Microsoft Excel software. Several miRNA reads were found to contain sequencing errors, typically starting from nucleotide 18-25 that were possibly due to the procedure of library preparation and/or pyrosequencing. Most errors were poly(A) insertions at the 3'-end of the reads. Therefore, those reads that were fully complementary to a known miRNA from nucleotide 1-18 but had additional A (adenosine) insertions at the 3'-end were re-annotated as miRNAs.

### 1.13. Luciferase assays

To investigate miRNA effects on reporter constructs, T98G cells were co-transfected with antisense 2'O methyl-oligonucleotides (100 nM final concentration) and pMIR-RL constructs (200 ng per well) in 48-well plates, using Lipofectamine 2000 (from Invitrogen). 24 h after transfection, cells were lysed in passive lysis buffer (from Promega). Luciferase activities were measured on a Mithras LB 940 luminometer (from Berthold Technologies, Germany). Luciferase substrate reagents were purchased from PJK Cryosystems (Germany). All samples were assayed in 4-6 replicates. Firefly/*Renilla* luminescence ratios for individual pMIR-RL 3'-UTR reporter constructs were normalized to corresponding ratios of the empty pMIR-RL plasmid.

### 1.14. Western Blotting and Northern Blotting

Western Blotting was performed as previously described (Hock, J. et al. (2007) EMBO Rep. 8, 1052-1060). The following antibodies (dilutions) were used: anti-mouse-HRP (1:5000), anti-rabbit-HRP (1:5000), mouse-anti-β-Actin AC15 (1:10000), mouse-anti-Tuj1 (1:1000), rabbit-anti-GFAP (1:2000). Northern blotting using either 2'O methyl oligonucleotide or DNA probes complementary to the miRNA of interest was essentially performed as previously described (Lagos-Quintana, M. et al. (2001) Science 294, 853-858) except that all hybridization and wash steps were carried out at 45°C.

### 1.15 Lactate dehydrogase (LDH) release assay

LDH assay was performed using the cytotoxicity detection kit (from Roche Diagnostics), according to the manufacturer's instructions. R11 cells were transfected with antagomirs (cf. above), cultured for 7 d and transfected again. Lactate dehydrogenase activity in the supernatant was measured 48 hrs after the second transfection.

### 1.16 Clonogenicity assays

R11 cells were transfected in 6-well plates with 2'O methyl oligonucleotides complementary to the miRNA of interest, cultured for 7 d, and transfected again. After another 7 d, cells were transferred from each well in a 6-well plate to one 48 well plate. Neurosphere-like clones were counted 4 weeks after the second transfection. The number of clones per well was normalized to the values obtained for control-transfected cells.

### 1.17. Statistical analysis

Experiments were performed in three biological replicates, unless stated otherwise. Mean values and standard error of the mean (SEM) were calculated from all biological replicates. Error bars display +/- SEM. Significance was assessed from biological replicates using two-sided Student's t-tests for unequal sample variance. P-values of < 0.05 were considered as significant.

### Example 2: miRNA expression profiles of CD133⁺ and CD133⁻ glioblastoma cells

### 2.1. Sorting/separation of CD133⁺ and CD133⁻ glioblastoma cell fractions

Fig. 1(A) schematically illustrates the experimental protocol employed for cell sorting. Primary human glioblastoma cell lines were incubated with ananti-CD133-phycoerythrin (PE) antibody and separated by fluorescence-assisted cell sorting (FACS) into CD133-positive and CD133-negative cell fractions. Subsequently, RNA was isolated and used to generate small RNA libraries, followed by 454 pyro-sequencing. A typical FACS profile of the primary glioblastoma cell line R11 is shown in Fig. 1(B). The dot-plot shows CD133-PE fluorescence on the x-axis and fluorescein isothiocyanate (FITC) on the y-axis as a control for autofluorescence. The regions indicate CD133-negative and CD133-positive cells, respectively. The CD133-positive (CD133⁺) and CD133-negative (CD133⁻) cell fractions were determined to be 11.8% and 86.9%, respectively.

Flow cytometry represents an efficient means for separating CD133-positive and CD133-negative cells (Fig. 5). R11 glioblastoma cells were analyzed as described above. Fig. 5(A) represents an exemplary flow cytometry analysis of unstained cells (left panel) and cells stained with anti-CD133-PE (right panel). Fig. 5(B) depicts an analysis, wherein after FACS separation, CD133-negative (left panel) and CD133-positive cell fractions (right panel) were re-analyzed by flow cytometry.

### 2.2. miRNA expression profiling

Expression profiling was performed s described above. Fig. 1(C) shows a representation of the most abundant miRNAs in the libraries of CD133-negative and CD133-positive cells. The y-axis shows log2 values of the relative miRNA abundance in CD133-positive cells vs. CD133-negative cells. Thus, miRNAs that are enriched in CD133-positive cells are displayed above the 0-axis. The size of the dots corresponds to the abundance of each miRNA in both libraries.

The results obtained revealed that hsa-miR-17-5p, hsa-miR-9*, hsa-miR-106b, hsa-miR-15b, hsa-miR-151-5p, hsa-miR-320, hsa-miR-23b, hsa-miR-25, hsa-miR-9, hsa-miR-191, hsa-miR-15a, hsa-miR-103, and hsa-miR-16 are enriched in CD133-positive cells, whereas hsa-miR-221, hsa-miR-222, hsa-miR-27a, hsa-miR-21, hsa-miR-26a, hsa-miR-23a, and hsa-miR-27b are enriched in CD133-negative cells. The sequences of these miRNAs are listed in Table 1, the sequences of the respective precursor molecules in Table 2.

The above results were confirmed by means of Northern blot analysis. The R28 primary glioblastoma cell line was separated into CD133-positive and CD133-negative cell fractions as described above in section 2.1. RNA was extracted and analyzed for the presence of miR-17-5p, hsa-miR-9*, hsa-miR-106b, and hsa-miR-9, all of them enriched in CD133-positive cells. U6 snRNA was used as loading control, Ile tRNA as a standard. All four miRNAs analyzed were significantly enriched in CD133-positive glioblastoma cells (Fig. 1(D)).

In a second analysis, primary glioblastoma cell lines R20, R28, R40, R44, and R52 were separated as described above and analyzed for the presence of hsa-miR-9 (upper panel) and hsa-miR-9* (lower panel) by quantitative PCR (qPCR). miRNA levels were normalized to U6 snRNA levels, and values for CD133-negative cell fractions were normalized to 1. hsa-miR-9 was enriched in the CD133-positive cell fractions of all five cell lines tested., with the highest level in R28. hsa-miR-9* was enriched in the CD133-positive cell fractions of R20, R28, R40, and R52, again with the highest level in R28. However, in the cell line R44 almost no miRNA could be detected in the CD133⁺ fraction. So far, the reason for this apparently inconsistent result has not been resolved but may likely result from experimental errors.

### Example 3: Inhibition of hsa-miR-9 and hsa-miR-9* expression impairs self-renewal and proliferation of human glioblastoma cells

### 3.1. Effect on cell number/cell survival

R11 cells were transfected twice with antagomirs (based on the antisense oligonucleotides described in section 1.3. above) and seeded to 48 well plates. Neurosphere-like colonies were counted 4 weeks after transfection. Transfection with hsa-miR-9 antisense completely blocked the formation of colonies, whereas transfection with hsa-miR-9* antisense resulted only in the formation of very few colonies. Transfection with hsa-miR-17-5p antisense and hsa-miR-106b resulted in less cell colonies than in the control, but the extent was much lower than with the other antagomirs (Fig. 2(A)).

In order to analyze the selectivity of the above results, cell survival rates were determined (Fig. 2(B)). R11 cells were transfected twice with antagomirs as described above (cf. section 1.15), and cell survival was assessed via measuring lactate dehydrogenase (LDH) activity in the supernatant 48 hours after the second transfection. Cell lysis with 1% Triton X-100 was used as positive control for cytotoxicity. Both hsa-miR-9 antisense and hsa-miR-9* antisense did not have any major impact on cell survival. The results were comparable to the positive control.

### 3.2. Influence of number of passages

In order to test any influence of the number of passages on efficacy of the antagomirs, single colonies of R11 cells that grew after the above treatments (cf. Fig. 2(A)) were picked, trypsinized and passaged to wells in a 48 well plate. Trypsinization efficiency was monitored by light microscopy. Cells were grown for 10 days, and the fraction of clones, which formed new clones was quantified (Fig. 2(C), left panel). The colonies quantified were picked and passaged again, as described above (Fig. 2(C), right panel). For both hsa-miR-9 antisense and hsa-miR-9* antisense the inhibitory effect was more pronounced after the second passage.

### 3.3. Effect on histone target mRNAs

In a further analysis, R11 cells were transfected with antagomirs as described above, RNA was isolated and the expression of the seven histone genes (i.e. HIST1H1E, HIST1H2A, HIST1H2BJ, HIST1H3D, HIST1H4G, HIST2H2AC, and HIST3H2BB) was assessed by qPCR. mRNA levels were normalized to GAPDH mRNA levels and to control samples. For all seven target histone genes, the transfection of both hsa-miR-9 antisense and hsa-miR-9* antisense resulted in a down-regulation of the respective histone mRNA levels, with HIST1H4G mRNA showing the highest extent of repression (Fig. 2(D)).

### 3.4. Specificity of inhibition

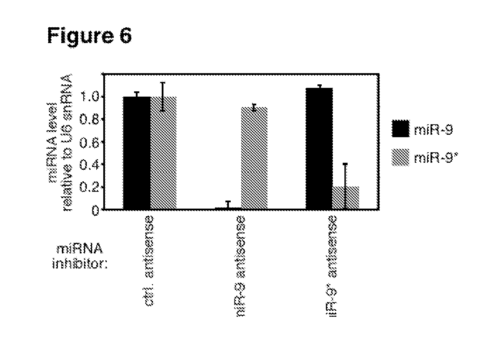

Finally, hsa-miR-9 and hsa-miR-9* levels in glioblastoma cells can be independently depleted by using 2'O methyl antisense oligonucleotides (Fig. 6). R11 cells were transfected twice with antagomirs to hsa-miR-9 and hsa-miR-9*, respectively. RNA was isolated and the corresponding miRNA levels were analyzed by qPCR relative to U6 RNA expression and to control antisense-transfected samples. Transfection of hsa-miR-9 antisense resulted in an almost complete depletion of hsa-miR-9 levels in the R11 cells, while hsa-miR-9* levels remained almost unchanged. *Vice versa,* transfection of hsa-miR-9* antisense resulted in an about 80% reduction of hsa-miR-9* levels in the R11 cells, while hsa-miR-9* levels remained unchanged.

### Example 4: Inhibition of hsa-miR-9 and hsa-miR-9* expression reduces the pool of CD133⁺ glioblastoma cells, enhances expression of the neuronal marker protein Tuj1 and reduces expression of the Wnt target gene WISP1.

### 4.1. Effect on CD133-positive cell fraction

R11 cells were transfected with hsa-miR-9 or hsa-miR-9* antagomirs for 2 days and analyzed for CD133 expression by flow cytometry as described above. Fig. 3(A) shows the size of the CD133-positive cell fractions relative to the control sample. The respective inhibition_of hsa-miR-9 or hsa-miR-9* expression reduces the pool of CD133⁺ glioblastoma cells by about 50%.

### 4.2. Effects on Tuji and WISP1 expression

After transfection with hsa-miR-9 or hsa-miR-9* antagomirs for 2 days, R11 cells were lysed and analyzed for the expression of neuronal class III β-tubulin (Tuj1) and glial fibrillary acidic protein (GFAP) by Western blotting. β-Actin was used as loading control. For both antagomirs a significant upregulation of Tuj1 expression was observed, GFAP expression remained essentially unchanged (Fig. 3(B)). Furthermore, RNA was isolated from the transfected R11 cells, and levels of WNT1-inducible signaling pathway protein 1 (WISP1) mRNA were quantified relative to GAPDH mRNA by qPCR as described above. Transfection with both antagomirs resulted in a significant reduction of WISP1 gene expression by about 60% for the hsa-miR-9 antagomir and about 50% for the hsa-miR-9* antagomir (Fig. 3(C)).

These results demonstrate that the inhibition of hsa-miR-9 and hsa-miR-9* in glioblastoma cell lines reduces the pool of CD133⁺ cells. Importantly, as mentioned above, in neuronal and/or glial tumors cancer stem cells seem to be restricted to this cell fraction. Furthermore, the expression of both miRNAs, which have been shown to be specific for neuronal tissues, is inversely correlated with the expression of a neuronal marker protein. And finally, the inhibition of these miRNAs appears to result in the down-regulation of the Wnt/β-catenin signaling cascade, which is associated with many types of cancer and also implicated in cell maintenance and self-renewal (reviewed, e.g., in Taipale, J. and Beachy, P.A. (2001) *supra*).

Hence, inhibition of hsa-miR-9 and hsa-miR-9* expression in fact appears to impair proliferation and self-renewal of cancer stem cells.

### Example 5: hsa-miR-9 and hsa-miR-9* repress the expression of Onecut1 and Onecut2

Fig. 4(A) depicts a schematic representation of the Onecut1 and Onecut2 3'-UTRs including miRNA seed matches, which are predicted or not predicted as miRNA binding sites.

T98G human glioma cells were co-transfected with hsa-miR-9 or hsa-miR-9* antagomirs and dual luciferase reporter constructs carrying the indicated 3'-UTRs (cf. section 1.4. above) fused to the firefly luciferase open reading frame (orf). Luminescence was measured 24 hrs post transfection, and *firefly*/*Renilla* ratios were normalized to control antagomir and to control plasmid transfections (Fig. 4(B)). Both antagomirs repressed the expression of all three 3'-UTRs tested.

In a second analysis, R11 cells were transfected with hsa-miR-9 or hsa-miR-9* antagomirs for 2 days. RNA was isolated, and Onecut1 and Onecut2 mRNA expression was quantified by qPCR relative to β-actin mRNA levels. Onecut2 mRNA expression was increased after transfection with either antagomir, whereas Onecut1 mRNA expression remained essentially unchanged (Fig. 4(C)). Fig. 4(D) shows Onecut2 mRNA levels in CD133-positive cells, relative to CD133-negative cells and to β-actin mRNA.

The present invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by embodiments and optional features, modifications and variations of the inventions embodied therein may be resorted to by those skilled in the art, and that such modifications and variations are considered to be within the scope of this invention.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

**TABLE 1: Nucleic acid sequences of the mature miRNAs disclosed herein.**

| **miRNA** | **Sequence (5' → 3')** |
|---|---|
| hsa-miR-9 | UCUUUGGUUAUCUAGCUGUAUGA |
| hsa-miR-9* | AUAAAGCUAGAUAACCGAAAGU |
| hsa-miR-17-5p | CAAAGUGCUUACAGUGCAGGUAG |
| hsa-miR-106b | UAAAGUGCUGACAGUGCAGAU |
| hsa-miR-15b | UAGCAGCACAUCAUGGUUUACA |
| hsa-miR-151-5p | UCGAGGAGCUCACAGUCUAGU |
| hsa-miR-320 | AAAAGCUGGGUUGAGAGGGCGA |
| hsa-miR-23b | AUCACAUUGCCAGGGAUUACC |
| hsa-miR-25 | CAUUGCACUUGUCUCGGUCUGA |
| hsa-miR-191 | CAACGGAAUCCCAAAAGCAGCUG |
| hsa-miR-15a | UAGCAGCACAUAAUGGUUUGUG |
| hsa-miR-103 | AGCAGCAUUGUACAGGGCUAUGA |
| hsa-miR-16 | UAGCAGCACGUAAAUAUUGGCG |
| hsa-miR-221 | AGCUACAUUGUCUGCUGGGUUUC |
| hsa-miR-222 | AGCUACAUCUGGCUACUGGGU |
| hsa-miR-27a | UUCACAGUGGCUAAGUUCCGC |
| hsa-miR-21 | UAGCUUAUCAGACUGAUGUUGA |
| hsa-miR-26a | UUCAAGUAAUCCAGGAUAGGCU |
| hsa-miR-23a | AUCACAUUGCCAGGGAUUUCC |
| hsa-miR-27b | UUCACAGUGGCUAAGUUCUGC |

**TABLE 2: Nucleic acid sequences of the miRNA precursors disclosed herein.**

| **miRNA** | **Sequence (5' → 3')** |
|---|---|
| hsa-miR-9 | CGGGGUUGGUUGUUAUCUUUGGUUAUCUAGCUGUAUGAGUGGUG UGGAGUCUUCAUAAAGCUAGAUAACCGAAAGUAAAAAUAACCCCA |
| | GGAAGCGAGUUGUUAUCUUUGGUUAUCUAGCUGUAUGAGUGUAUU GGUCUUCAUAAAGCUAGAUAACCGAAAGUAAAAACUCCUUCA |
| | GGAGGCCCGUUUCUCUCUUUGGUUAUCUAGCUGUAUGAGUGCCAC AGAGCCGUCAUAAAGCUAGAUAACCGAAAGUAGAAAUGAUUCUCA |
| hsa-miR-9* | CGGGGUUGGUUGUUAUCUUUGGUUAUCUAGCUGUAUGAGUGGUG UGGAGUCUUCAUAAAGCUAGAUAACCGAAAGUAAAAAUAACCCCA |
| | GGAAGCGAGUUGUUAUCUUUGGUUAUCUAGCUGUAUGAGUGUAUU GGUCUUCAUAAAGCUAGAUAACCGAAAGUAAAAACUCCUUCA |
| | GGAGGCCCGUUUCUCUCUUGGUUAUCUAGCUGUAUGAGUGCCAC AGAGCCGUCAUAAAGCUAGAUAACCGAAAGUAGAAAUGAUUCUCA |
| hsa-miR-17-5p | GUCAGAAUAAUGUCAAAGUGCUUACAGUGCAGGUAGUGAUAUGUG CAUCUACUGCAGUGAAGGCACUUGUAGCAUUAUGGUGAC |
| hsa-miR-106b | CCUGCCGGGCUAAAGUGCUGACAGUGCAGAUAGUGGUCCUCUC CGUGCUACCGCACUGUGGGUACUUGCUGCUCCAGCAGG |
| hsa-miR-15b | UUGAGGCCUUAAAGUACUGUAGCAGCACAUCAUGGUUUACAUGCU ACAGUCAAGAUGCGAAUCAUUAUUUGCUGCUCUAGAAAUUUAAGG AAAUUCAU |
| hsa-miR-151-5p | UUUCCUGCCCUCGAGGAGCUCACAGUCUAGUAUGUCUCAUCCCCU ACUAGACUGAAGCUCCUUGAGGACAGGGAUGGUCAUACUCACCUC |
| hsa-miR-320 | GCUUCGCUCCCCUCCGCCUUCUCUUCCCGGUUCUUCCCGGAGUCG GGAAAAGCUGGGUUGAGAGGGCGAAAAGGAUGAGGU |
| hsa-miR-23b | CUCAGGUGCUCUGGCUGCUUGGGUUCCUGGCAUGCUGAUUUGUGA CUUAAGAUUAAAAUCACAUUGCCAGGGAUUACCACGCAACCACGAC CUUGGC |
| hsa-miR-25 | GGCCAGUGUUGAGAGGCGGAGACUUGGGCAAUUGCUGGACGCUG CCCUGGGCAUUGCACUUGUCUCGGUCUGACAGUGCCGGCC |
| hsa-miR-191 | CGGCUGGACAGCGGGCAACGGAAUCCCAAAAGCAGCUGUUGUCUC CAGAGCAUUCCAGCUGCGCUUGGAUUUCGUCCCCUGCUCUCCUGC CU |
| hsa-miR-15a | CCUUGGAGUAAAGUAGCAGCACAUAAUGGUUUGUGGAUUUUGAAA AGGUGCAGGCCAUAUUGUGCUGCCUCAAAAAUACAAGG |
| hsa-miR-103 | UACUGCCCUCGGCUUCUUUACAGUGCUGCCUUGUUGCAUAUGGAU CAAGCAGCAUUGUACAGGGCUAUGAAGGCAUUG |
| | UUGUGCUUUCAGCUUCUUUACAGUGCUGCCUUGUAGCAUUCAGGU CAAGCAGCAUUGUACAGGGCUAUGAAAGAACCA |
| hsa-miR-16 | GUCAGCAGUGCCUUAGCAGCACGUAAAUAUUGGCGUUAAGAUUCU AAAAUUAUCUCCAGUAUUAACUGUGCUGCUGAAGUAAGGUUGAC- |
| | GUUCCACUCUAGCAGCACGUAAAUAUUGGCGUAGUGAAAUAUAUA UUAAACACCAAUAUUACUGUGCUGCUUUAGUGUGAC |
| hsa-miR-221 | UGAACAUCCAGGUCUGGGGCAUGAACCUGGCAUACAAUGUAGAUU UCUGUGUUCGUUAGGCAACAGCUACAUUGUCUGCUGGGUUUCAGG CUACCUGGAAACAUGUUCUC |
| hsa-miR-222 | GCUGCUGGAAGGUGUAGGUACCCUCAAUGGCUCAGUAGCCAGUGU AGAUCCUGUCUUUCGUAAUCAGCAGCUACAUCUGGCUACUGGGUC UCUGAUGGCAUCUUCUAGCU |
| hsa-miR-27a | CUGAGGAGCAGGGCUUAGCUGCUUGUGAGCAGGGUCCACACCAAG UCGUGUUCACAGUGGCUAAGUUCCGCCCCCCAG |
| hsa-miR-21 | UGUCGGGUAGCUUAUCAGACUGAUGUUGACUGUUGAAUCUCAUGG CAACACCAGUCGAUGGGCUGUCUGACA |
| hsa-miR-26a | GUGGCCUCGUUCAAGUAAUCCAGGAUAGGCUGUGCAGGUCCCAAU GGGCCUAUUCUUGGUUACUUGCACGGGGACGC |
| | GGCUGUGGCUGGAUUCAAGUAAUCCAGGAUAGGCUGUUUCCAUCU GUGAGGCCUAUUCUUGAUUACUUGUUUCUGGAGGCAGCU |
| hsa-miR-23a | GGCCGGCUGGGGUUCCUGGGGAUGGGAUUUGCUUCCUGUCACAA AUCACAUUGCCAGGGAUUUCCAACCGACC |
| hsa-miR-27b | ACCUCUCUAACAAGGUGCAGAGCUUAGCUGAUUGGUGAACAGUGA UUGGUUUCCGCUUUGUUCACAGUGGCUAAGUUCUGCACCUGAAGA GAAGGUG |

## Claims

1. Diagnostic kit of molecular markers for identifying one or more cancer stem cells, the kit comprising a plurality of nucleic acid molecules, each nucleic acid molecule encoding a microRNA sequence, wherein one or more of the plurality of nucleic acid molecules are differentially expressed in the cancer stem cells and in one or more control cells, and wherein the one or more differentially expressed nucleic acid molecules together represent a nucleic acid expression signature that is indicative for the presence of cancer stem cells.

2. The kit of claim 1, wherein the cancer stem cells are CD133-positive, and wherein the control cells are CD133-negative cancer cells.

3. The kit of claim 1 or 2, wherein the cancer stem cells are derived from neuronal and/or glial tumors, preferably from gliobastoma.

4. The kit of any of claims 1 to 3, wherein the nucleic acid expression signature comprises at least three, preferably at least five, and more preferably at least eight nucleic acid molecules.

5. The kit of any of claims 1 to 4, wherein the nucleic acid expression signature comprises nucleic acid molecules encoding microRNA sequences selected from the group consisting of hsa-miR-9 and hsa-miR-9*.

6. The kit of claim 5, wherein the expression of any of the nucleic acid molecules encoding hsa-miR-9 and hsa-miR-9* is up-regulated in the one or more cancer stem cells compared to the one or more control cells.

7. The kit of claim 5 or 6, wherein the nucleic acid expression signature further comprises any one or more nucleic acid molecules encoding microRNA sequences selected from the group consisting of hsa-miR-17-5p, hsa-miR-106b, hsa-miR-15b, hsa-miR-151-5p, hsa-miR-320, hsa-miR-23b, hsa-miR-25, hsa-miR-191, hsa-miR-15a, hsa-miR-103, hsa-miR-16, hsa-miR-221, hsa-miR-222, hsa-miR-27a, hsa-miR-21, hsa-miR-26a, hsa-miR-23a, and hsa-miR-27b.

8. The kit of claim 7, wherein the expression of any of the nucleic acid molecules encoding hsa-miR-17-5p, hsa-miR-106b, hsa-miR-15b, hsa-miR-151-5p, hsa-miR-320, hsa-miR-23b, hsa-miR-25, hsa-miR-191, hsa-miR-15a, hsa-miR-103, and hsa-miR-16 is up-regulated and the expression of any of the nucleic acid molecules hsa-miR-221, hsa-miR-222, hsa-miR-27a, hsa-miR-21, hsa-miR-26a, hsa-miR-23a, and hsa-miR-27b is down-regulated in the in the one or more cancer stem cells compared to the one or more control cells.

9. The kit of claim 7 or 8, wherein the nucleic acid expression signature comprises nucleic acid molecules encoding hsa-miR-17-5p, hsa-miR-106b, hsa-miR15b, hsa-miR-221, hsa-miR-222, hsa-miR-27a, and hsa-miR-21.

10. Method for identifying one or more cancer stem cells, preferably CD133-positive cancer stem cells, the method comprising:
(a) determining in the one or more cancer stem cells the expression levels of a plurality of nucleic acid molecules, each nucleic acid molecule encoding a microRNA sequence;
(b) determining the respective expression levels of the plurality of nucleic acid molecules in one or more control cells; and
(c) identifying from the plurality of nucleic acid molecules one or more nucleic acid molecules that are differentially expressed in the target and control cells by comparing the respective expression levels obtained in steps (a) and (b),
wherein the one or more differentially expressed nucleic acid molecules together represent a nucleic acid expression signature, as defined in any of claims 1 to 9, that is indicative for the presence of cancer stem cells.

11. The method of claim 10, wherein the cancer stem cells are CD133-positive and the control cells are CD133-negative cancer cells, and wherein the method further comprises separating the CD133-positive and CD133-negative cells prior to performing step (a).

12. Method for preventing the proliferation and/or self-renewal of one or more cancer stem cells, preferably CD133-positive cancer stem cells, the method comprising:
(a) identifying in the one or more cancer stem cells a nucleic acid expression signature by using a method as defined in claim 10 or 11; and
(b) modifying in the one or more cancer stem cells the expression of one or more nucleic acid molecules encoding a microRNA sequence that is/are comprised in the nucleic acid expression signature in such way that the expression of a nucleic acid molecule whose expression is up-regulated in the one or more cancer stem cells is down-regulated and the expression of a nucleic acid molecule whose expression is down-regulated in the one or more cancer stem cells is up-regulated.

13. Pharmaceutical composition for preventing the proliferation and/or self-renewal of one or more cancer stem cells, preferably CD133-positive cancer stem cells, the composition comprising one or more nucleic acid molecules, each nucleic acid molecule encoding a sequence that is at least partially complementary to a microRNA sequence encoded by a nucleic acid molecule whose expression is up-regulated in the one or more cancer stem cells, as defined in any of claims 1 to 9, and/or that corresponds to a microRNA sequence encoded by a nucleic acid molecule whose expression is down-regulated in the one or more cancer stem cells, as defined in any of claims 1 to 9.

14. The pharmaceutical composition of claim 13, wherein the cancer stem cells are derived from neuronal and/or glial tumors, preferably from gliobastoma.

15. Use of the pharmaceutical composition of claim 13 or 14 for the manufacture of a medicament for the prevention and/or treatment of cancer, preferably of neuronal and/or glial cancers.
